(19) Europäisches Patentamt
European Patent Office
Office européen des brevets

(11) EP 3 246 413 A1

(12) EUROPEAN PATENT APPLICATION

(43) Date of publication:
22.11.2017 Bulletin 2017/47

(51) Int Cl.:
*C12Q 1/68* (2006.01)

(21) Application number: 16170625.4

(22) Date of filing: 20.05.2016

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
MA MD

(71) Applicants:
- Klinikum rechts der Isar der Technischen Universität München
  81675 München (DE)
- Helmholtz Zentrum München Deutsches Forschungszentrum für Gesundheit und Umwelt GmbH
  85764 Neuherberg (DE)

(72) Inventors:
- SCHMITT, Manfred
  80977 München (DE)
- NAPIERALSKI, Rudolf
  81735 München (DE)
- SCHÜREN, Elisabeth
  82327 Tutzing (DE)
- AUBELE, Michaela
  85614 Kirchseeon (DE)
- ABSMAIER, Magdalena
  80801 München (DE)
- GROSS, Eva
  82110 Germering (DE)
- SCHUSTER, Tibor
  Montreal, QC H2W 2C5 (CA)
- HÖFLER, Heinz
  81675 München (DE)
- KIECHLE, Marion
  81545 München (DE)

(74) Representative: Engelhard, Markus
Boehmert & Boehmert
Anwaltspartnerschaft mbB
Pettenkoferstrasse 22
80336 München (DE)

# (54) A METHOD FOR PREDICTING A BREAST CANCER PATIENT'S RESPONSE TO ANTHRACYCLINE TREATMENT

(57) The present invention relates to a method for predicting a breast cancer patient's response to anthracycline treatment.

EP 3 246 413 A1

## Description

The present invention relates to a method for predicting a breast cancer patient's response to anthracycline treatment.

In Germany, presently there are 75,000 diagnosed cases of breast cancer. In the year 2012, globally, 1.7 million cases of breast cancer have been diagnosed. According to estimates of the International Agency for Research and Cancer of the World Health Organization, incidents of breast cancer have increased by 20 % since 2008, and mortality has increased by 14 %. Prognosis factors provide information about the probability of the further course of the disease (disease recurrence, absence of disease recurrence and overall survival), whereas prediction factors provide information about the likelihood of a patient responding to a specific therapy. Clinically used histomorphological prognostic factors comprise the of axillary lymph node status, tumor size and histological degree of differentiation as well as invasion into lymph or blood vessels, tumor staging, various molecular markers such as the estrogen receptor (ER), progesterone receptor (PR), urokinase-type plasminogen activator (uPA), plasminogen activator inhibitor type 1 (PAI-1), and prognostic multigene marker panels such as Oncotype DX®, Mammaprint® or the Endopredict Test ®. Currently, there is no test kit available commercially which would provide information about the responsiveness of a patient with respect to specific chemotherapeutic agents.

Approximately 15 % of breast cancers can be classified as triple-negative breast cancer (TNBC). These are known for their poor outcome, early disease onset, and high aggressiveness.[5, 6] TNBC is characterised by the lack of estrogen receptor (ER), progesterone receptor (PgR) and absence of amplification of human epidermal growth factor receptor 2 (HER2).[10, 40] Consequently, patients suffering from TNBC do not derive benefit from established therapies targeting these receptors. Despite its definition, gene expression profiling revealed that TNBC constitutes a very heterogeneous disease in need for further classification, with overlapping characteristics with e.g. basal-like breast cancer and claudin-low intrinsic subtype.[28, 29, 33] At present, chemotherapy remains one of the most common therapeutic approaches in TNBC and both in neoadjuvant and adjuvant settings, anthracycline- and taxane-based regimens are most frequently applied.[38] They can lead to severe side-effects though as e.g. cytotoxicity of anthracyclines can cause cardiotoxicity and secondary leukemia.[16] Furthermore, an ambiguity concerning the optimal chemotherapy regimen for TNBC remains as contradictory results regarding the benefit derived from anthracycline treatment were revealed.[27] Due to the poor outcome and unavailability of targeted therapies, further characterisation and classification of TNBC according to prognostic and/or predictive markers could improve the currently poor outcome of affected patients.

Relatively new approaches involve the field of epigenetics. DNA methylation plays an important role in controlling gene activity and nucleus architecture, generally promoting chromatin condensation and transcriptional inactivity.[15] In breast cancer, genome-wide DNA methylation profiling assays revealed about 2,000 hypermethylation and 1,500 hypomethylation events [26], including several genes regulating cell invasion, cell-cycle, apoptosis, DNA repair and metastasis.[15]

Due to the severity of TNBC and due to the severe side-effects of currently applied chemotherapy, it would be desirable to have a methodology at hand which allows to predict the likelihood of success of a chemotherapy in a TNBC patient. More specifically, it was an object of the present invention to provide for a methodology that allows to predict a breast cancer patient's responsiveness to chemotherapy, in particular anthracycline chemotherapy. More specifically, it was an object of the present invention to provide for a methodology allowing the prediction of a TNBC patient's responsiveness to anthracycline treatment.

All these objects are solved by a method for predicting a triple-negative breast cancer (TNBC) patient's response to anthracycline-containing polychemotherapy, said method comprising:

i) contacting genomic DNA isolated from a biological sample of said TNBC patient with at least one reagent, or series of reagents, that distinguishes between methylated and non-methylated CpG dinucleotides;
ii) determining, based on such contacting of i), a methylation state of at least one CpG dinucleotide sequence of the paired-like homeodomain transcription factor 2 (PITX2) gene and/or of one or several regulatory sequences thereof within said biological sample, and
iii) making a prediction of said TNBC patient's response to anthracycline-containing polychemotherapy based on the determined methylation state.

In one embodiment, said polychemotherapy is adjuvant polychemotherapy.

In one embodiment, said prediction of said TNBC patient's response to anthracycline-containing polychemotherapy is based on whether the determined methylation state of said PITX2 gene and/or of one or several regulatory sequences thereof exceeds a defined threshold, wherein said prediction is made in terms of one or several parameters selected from disease-free survival (DFS), metastasis-free survival (MFS) and overall survival (OS), respectively, and wherein said prediction is negative, if the determined methylation state is equal to or does not exceed said threshold, and wherein said prediction is positive, if said determined methylation state does exceed said threshold.

In one embodiment, said contacting in step i) comprises contacting genomic DNA isolated from said biological sample obtained from said patient with at least one reagent, or series of reagents that distinguishes between methylated and non-methylated CpG dinucleotides within at least one target region of the genomic DNA, wherein the target region comprises or hybridizes under stringent conditions to a sequence of at least 16 contiguous nucleotides of the PITX2 gene and/or of regulatory sequences thereof, wherein said contiguous nucleotides comprise at least one CpG dinucleotide sequence.

In one embodiment, said one or more reagents comprise bisulfite, hydrogen sulfite, disulfite or combinations thereof.

In one embodiment, the step of determining the methylation state is performed by oligonucleotide hybridization analysis, methylation-sensitive single-nucleotide primer extension (Ms-SNuPE), sequencing, quantitative real time PCR or oligonucleotide array analysis.

In one embodiment, said determination of said methylation state in step ii) is or comprises the determination of a methylation score of said PITX2 gene or of one or several regulatory sequences thereof in said biological sample, and wherein prediction of said TNBC patient's response to said anthracycline-containing polychemotherapy is based on whether the methylation score in said biological sample exceeds a defined threshold methylation score, wherein said prediction is made in terms of one or several parameters selected from disease-free survival (DFS), metastasis-free survival (MFS) and overall survival (OS), and wherein said prediction is negative, if the methylation score of said sample is equal to or does not exceed said defined threshold methylation score, and wherein said prediction is positive, if said methylation score of said sample does exceed said defined threshold methylation score,
wherein said methylation score in said biological sample is determined according to the formula:

$$\text{methylation score in biological sample} = \frac{100}{1+2^{(CT\ methylated - CT\ unmethylated)}}$$

wherein "CT methylated" is the cycle threshold value of methylated PITX2 and/or of methylated regulatory sequence(s) thereof,
and wherein "CT unmethylated" is the cycle threshold value of unmethylated PITX2 and/or of unmethylated regulatory sequence(s) thereof.

In one embodiment, the method comprises the steps:

i') isolating genomic DNA from a biological sample taken from said patient and treating said genomic DNA, or a fragment thereof, with one or more reagents, in order to convert 5-position unmethylated cytosine bases to uracil or to another base that is detectably dissimilar to cytosine in terms of hybridization properties, thus producing treated genomic DNA; contacting said treated genomic DNA or said treated fragment thereof, with an amplification enzyme and at least two primers comprising, in each case, a contiguous sequence of at least 16 nucleotides in length that is complementary to, or hybridizes under stringent conditions to a sequence of the PITX2 gene and/or to one or several of its regulatory sequences thereof, and/or to complements thereof, wherein said at least two primers hybridize to said sequence(s) only if such sequence(s) has(have) been treated with said one or more reagents, such that the treated DNA or a fragment thereof is amplified to produce one or more amplificates, and wherein during amplification there are additionally at least two PITX2-specific probes present that distinguish between methylated and unmethylated PITX2 sequences, said at least two PITX2-specific probes producing two signals being indicative of methylated and unmethylated PITX2 sequences, respectively, said signals being proportional to the amount of methylated und unmethylated PITX2-sequences present in said biological sample, respectively,

ii') determining, based on said signals of methylated und unmethylated PITX2-sequences a methylation score of said PITX2 gene in said biological sample,
wherein said methylation score in said biological sample is determined according to the formula

$$\text{methylation score in biological sample} = \frac{100}{1+2^{(CT\ methylated - CT\ unmethylated)}}$$

wherein "CT methylated" is the cycle threshold value of methylated PITX2 and/or of methylated regulatory sequence(s) thereof,
and wherein "CT unmethylated" is the cycle threshold value of unmethylated PITX2 and/or of unmethyated regulatory sequence(s) thereof, and

iii') making a prediction of said TNBC patient's response to anthracycline-containing polychemotherapy based on said determined methylation score in said biological sample, and wherein prediction of said TNBC patient's response to said anthracycline-containing polychemotherapy is based on whether the methylation score in said biological sample exceeds a defined threshold methylation score, wherein said prediction is made in terms of one or several parameters selected from disease-free survival (DFS), metastasis-free survival (MFS) and overall survival (OS), and wherein said prediction is negative, if the methylation score of said sample is equal to or does not exceed said defined threshold methylation score, and wherein said prediction is positive, if said methylation score of said sample is does exceed said defined threshold methylation score.

In one embodiment, the method further comprises

iv) determining a suitable treatment regimen for said patient, wherein such suitable treatment regimen for said patient is a treatment with polychemotherapy including one or several anthracyclines if said prediction of step iii) or iii') is positive, and said suitable treatment regimen is a therapy excluding anthracycline treatment, if said prediction is negative.

In one embodiment, said defined threshold methylation score is in the range of from 4 - 10, particularly in the range of from 5 - 8, more particularly 5- 7, even more particularly 6 - 7, most particularly approximately 6.35 +/-0.3.

In one embodiment, said biological sample is selected from the group consisting of breast cancer-derived tumor cell lines and breast cancer tissues, e.g. presurgical core biopsies, biopsies taken at time of primary surgery, circulating peripheral breast cancer tumor cells, tumor-afflicted lymph nodes, metastases, in particular in the frozen state or fixed with any fixative and then embedded in paraffin, bodily fluids such as nipple aspirate, blood, serum, plasma, urine or any other bodily fluid, and combinations thereof.

In one embodiment, the PITX2 gene has a sequence represented by SEQ ID NO:1 or SEQ ID NO:2.

In one embodiment, said sequence which said at least two primers are complementary to or hybridize thereto has a sequence represented by SEQ ID NO:3 and/or 4, and/or wherein said sequence of the PITX2 gene before treatment with said one or more reagent(s) has a sequence represented by SEQ ID NO:5, and/or wherein said at least two primers have sequences represented by SEQ ID NO: 6 and 7; and/or wherein said at least two PITX2-specific probes have sequences represented by SEQ ID NO:8 and SEQ ID NO:9.

In one embodiment, said at least two PITX2-specific probes are Taqman hydrolysis probes that distinguish between methylated and unmethylated PITX2 sequences, wherein a first Taqman hydrolysis probe is specific for methylated PITX2 sequence(s) and produces a first signal upon amplification of methylated PITX2 sequence(s), and wherein a second Taqman hydrolysis probe is specific for unmethylated PITX2 sequence(s) and produces a second signal different from said first signal, upon amplification of unmethylated PITX2 sequence(s), said first and said second signal preferably being fluorescence signals.

The objects of the present invention are also solved by the use of a PITX2 gene, a PITX2 regulatory sequence, or a stretch of at least 16 contiguous nucleotides of the foregoing sequences, or of a complementary sequence thereto or of a sequence that hybridizes under stringent conditions to any of the foregoing sequences, in a method for predicting a triple-negative breast cancer (TNBC) patient's response to anthracycline-containing adjuvant polychemotherapy according to the present invention.

In one embodiment, said PITX2 gene, said PITX2 regulatory sequence, said stretch of at least 16 contiguous nucleotides of the foregoing sequences, said complementary sequence thereto, or said sequence that hybridizes under stringent conditions to any of the foregoing sequences, is selected from any of the sequences represented by SEQ ID NO:1 - 9.

The present inventors have surprisingly found that, as opposed to non-triple-negative breast cancer (non-TNBC), a low DNA methylation state in the PITX2 gene, and thus a hypomethylation thereof, means that such a patient is likely to be non-responsive to anthracycline treatment. A TNBC patient thus diagnosed with a hypomethylation of the PITX2 gene is therefore likely not to respond to an anythracycline treatment and should therefore not be treated with anthracyclines, as this will have little or no positive effect on the patient's recovery. For such a patient, the treating physician will therefore choose another suitable treatment which does not involve the use of anthracyclines.

The term "methylation state", as used herein, is meant to refer to the degree of methylation present in a nucleic acid of interest. This may be expressed in absolute or relative terms, i. e. as a percentage or other numerical value or by comparison to another tissue and may be described as "hypermethylated", "hypomethylated" or as "having significantly similar or identical methylation status". A "methylation score" as used therein, is a continuous variable and is typically expressed as "percent methylation ratio" (PMR). The "methylation score" is typically calculated according to the formula $100/(1+2^{(CTmethylated-CTunmethylated)})$ wherein "CT methylated" is the cycle threshold value of methylated sequence of interest, and wherein "CT unmethylated" is the cycle threshold value of the unmethylated sequence of interest. Cycle threshold values are a standard entity in quantitative PCT and refer to the number of cycles required for the signal to exceed background level. The term is a standard term and is known to a person skilled in the art. Cycle threshold values are inversely proportional to the amount of target nucleic acid in the sample, i. e. the lower the CT value is, the greater the amount of target nucleic acid in the sample. A methylation score can be calculated with or without normalization against one or several housekeeping genes. If such normalization is involved, this is also sometimes refered to as "double delta CT analysis". In the experimental section that is described hereafter, cycle threshold values are determined automatically by the software of the quantitative PCR cycler. More specifically, in the experiments performed by the present inventors,

Cycle threshold values (CT) are determined automatically for the markers PITX2 methylated (FAM-Channel) and PITX2 unmethylated (VIC/HEX-Channel) separately by the qPCR cycler software by the course of the fluorescent signal readout during the PCR cycle program and including adaptive baseline correction and amplification-based threshold value (in the area of 5-60 % total fluorescence level, averaged over technical replicates). Data transformation of CT values in a methylation score or, synonymously herewith, percent methylation ratio (PMR) values is facilitated by a modified $2\exp^{\Delta CT}$ method for a duplex probe system with internal calibration and standardization.

The term "threshold" or "defined threshold methylation score", as used herein, is meant to refer to a specifically defined value or a specific (narrow) range of DNA methylation scores, which separates those TNBC patients likely to benefit from an anthracycline treatment from those TNBC patients which are not likely to benefit from such anthracycline treatment. If the methylation score of the PITX2 gene in a biological sample is equal or smaller than the defined threshold methylation score, then the patient will likely not benefit from an anthracycline treatment. According to one embodiment of the invention, such a defined threshold methylation score is in the range of from 4-10, preferably from 5-8, more preferably from 5-7, even more preferably from 6-7, most preferably around 6.35, e. g. 6.35 $\pm$ 0.3.

The term "PITX2 gene" refers to the paired-like homeodomain transcription factor 2. An example embodiment of such PITX2 gene has the sequence represented by SEQ ID NO: 1 and can be found e. g. as entry http://www.ncbi.nlm.nih.gov/nuccore/161086966 or as entry: http://www.ncbi.nlm.nih.gov/nucleotide?cmd=Retrieve&dopt=GenBank&list uids=13183092 GenBank: AF238048.1 (SEQ ID NO:2).

A preferred PITX2 sequence according to one embodiment of the invention is:

Entrez gene ID 5308; Amplicon length 144; Reference sequence (Ref Seq) ID NT_016354.18; Detected CpG in Ref Seq 3 CpG in 36106573 - 36106600 which is shown below as SEQ ID NO:5 and which specifies the location of 3 CpG dinucleotides detected with Taqman hydrolysis probes, e.g. SEQ ID NO: 8 or 9.

Exemplary Sequences in accordance with the present invention:

Bisulfite-converted PITX 2 sequence with a fully methylated status (SEQ ID NO:3) to which the Taqman probe for methylated sequences will anneal (e.g. SEQ ID NO:8):

gtaggggagggaagtagatgttagcgggtcgaagagtcgggagtcggagtcgggagagcgaaaggagaggggatttggcg
gggtatttaggagttaatcgaggagtaggagtacggattttattgtggaaaggaggattagaa

Bisulfite-converted PITX2 sequence with an unmethylated status (SEQ ID NO:4) to which the Taqman probe for unmethylated sequences will bind (SEQ ID NO:9):

gtaggggagggaagtagatgttagtgggttgaagagttgggagttggagttgggagagtgaaaggagaggggatttggtggggt
atttaggagttaattgaggagtaggagtatggattttattgtggaaaggaggattagaa

Genomic (non-bisulfite-converted PITX2 sequence (SEQ ID NO: 5):

gcaggggagggaagcagatgccagcgggccgaagagtcgggagccggagccgggagagcgaaaggagaggggacctg
gcggggcacttaggagccaaccgaggagcaggagcacggactcccactgtggaaaggaggaccagaa

Exemplary Primer and probe sequences in accordance with the present invention:

| | |
|---|---|
| PITX2-R | ttctaatcctcctttccacaataa (SEQ ID NO:6) |
| PITX2-F | gtaggggagggaagtagatgtt (SEQ ID NO:7) |
| PITX2-Pm | agtcggagtcgggagagcga (SEQ ID NO:8) |
| PITX2-Pu | agttggagttgggagagtgaaaggaga (SEQ ID NO:9) |

F: forward primer
R: reverse primer
Pm: methylated probe (Taqman Hdyrolysis-probe with FAM)
Pu: unmethylated probe (Taqman Hdyrolysis-probe with VIC or HEX)

The term "anthracyclines", as used herein, is meant to refer to a group of drugs obtained from Streptomyces bacteria, in particular Streptomyces peucitius. Examples of anthracyclines are daunorubicin, doxorubicin, epirubicin, idarubicin, valrubicin and mitoxantrone.

The term "PITX2 gene", as used herein, is meant to refer to both introns and exons of the PITX2 gene as well as to regulatory sequences of PITX2. For example, such term also includes a promoter of the PITX2 gene. The term "PITX2 sequence", as used herein, is meant to refer to any such sequence of PITX2. It may refer to partial sequences of the PITX2 gene as short as 16 contiguous nucleotides or more. A probe or primer or sequence that is "PITX2-specific" is meant to refer to any primer or probe that specifically binds to, is complementary to or hybridizes to a PITX2-gene.

The term "hybridizes to" is meant to refer to a scenario wherein a single-stranded nucleic acid sequence binds to or anneals to another single-stranded nucleic acid. In one embodiment, this is achieved under a variety of conditions, e. g. stringent conditions. A person skilled in the art is able to determine stringent conditions for nucleic acid hybridization.

For Array experiments: "Stringent hybridization conditions," as defined herein, involve hybridizing at 68°C in 5x SSC/5x Denhardt's solution/1.0% SDS, and washing in 0.2x SSC/0.1% SDS at room temperature, or involve the art-recognized equivalent thereof (e.g., conditions in which a hybridization is carried out at 60°C in 2.5 x SSC buffer, followed by several washing steps at 37°C in a low buffer concentration, and remains stable). Moderately stringent conditions, as defined herein, involve including washing in 3x SSC at 42°C, or the art-recognized equivalent thereof. The parameters of salt concentration and temperature can be varied to achieve the optimal level of identity between the probe and the target nucleic acid. Guidance regarding such conditions is available in the art, for example, by Sambrook et al., 1989, Molecular Cloning, A Laboratory Manual, Cold Spring Harbor Press, N.Y.; and Ausubel et al. (eds.), 1995, Current Protocols in Molecular Biology, (John Wiley & Sons, N.Y.) at Unit 2.10.

For Taqman qPCR systems the inventors would recommend hybridizing at 60°C in a commercial quantitative PCR buffer (e.g. Quantitect Probe PCR buffer from the Company Qiagen) including reaction buffer, dNTPs, Magnesium and TAQ Polymerase.

The term "triple-negative breast cancer (TNBC)", as used herein, is meant to refer to a type of breast cancer which is characterized by a lack of estrogen receptor (ER) and progesterone receptor (PgR) expression and a low expression of human epidermal growth factor receptor 2 (HER2). (preferably in accordance with a Dako IHC score 0-2, without amplification of the HER2 gene assessed by fluorescence in situ hybridisation according to the Sankt Gallen guidelines for breast cancer.)

The term "predicting a patient's response to a treatment", as used herein, is meant to refer to a prediction of the outcome of a particular treatment, if performed on a patient. Preferably, such a statement about the response or outcome of a particular treatment is made in terms of one or several parameters selected from disease-free survival (DFS), metastasis-free survival (MFS) and overall survival (OS).

The term "regulatory sequence of a gene", as used herein, is meant to refer to a sequence which affects the expression of a gene. Such a regulatory sequence may be located within, proximal or distal to said gene. A regulatory sequence includes, but is not limited to constitutive promoters, tissue-specific promoters, developmental-specific promoters, inducible promoters and the like. Promoter-regulatory elements may also include enhancer sequence elements that control transcriptional or translational efficiency of a gene.

The term "methylation" refers to the presence or absence of 5-methyl-cytosine (5-mCyt)" at one or a plurality of CpG dinucleotides within a DNA sequence.

The term "methylation state", as used herein, is meant to refer to the degree of methylation present in a nucleic acid of interest, e.g. the PITX2 gene. This may be expressed in absolute or relative terms, i.e. as a percentage or other numerical value or by comparison to another tissue and may therein be described as "hypermethylated, hypomethylated or as having significantly similar or identical methylation status".

The methylation score is a continuous variable (and is herein also sometimes referred to as "Percent methylation ratio"), Methylation state discerns between a high methylated (hypermethylated) or low methylated (hypomethylated) state for the PITX2 gene according to a specific cut off value (e.g. 6.35 PMR).

The term "Taqman hydrolysis probe" as used herein, is meant to refer to an oligonucleotide probe which is sequence specific and which has a dye attached to one end of the probe and a quencher at the other end of the probe. Typically, the dye is a fluorescent dye, and the quencher is a fluorescence quencher. In the context of embodiments of the present invention, the oligonucleotide probe is PITX2-specific, in particular specific for methylated PITX2 sequences or for unmethylated PITX2 sequences.

In the Taqman probe, the quencher molecule quenches the fluorescence emitted by the fluorophor via fluorescence resonance energy transfer, for as long as the fluorophor and the quencher are in proximity to each other. During amplification, the amplification enzyme, typically a Taq polymerase, degrades the oligonucleotide probe due to the exonuclease activity of the polymerase, and a degradation of the probe releases the dye, typically the fluorophor, from the probe, as a result of which the quenching no longer occurs. The signal detected is thus directed proportional to the number of amplificates which, in turn, is directly proportional to the number of copies originally present in the biological sample.

Typical examples of fluorophor that are used in Taqman hydrolysis probes are fluorescein derivatives (FAM), VIC dye, HEX dye, i. e. 5'-hexachloro-fluorescein).

The term "adjuvant" chemotherapy, in the context of breast cancer, is meant to refer to a chemotherapy that is performed after surgery or any other form of primary therapy. The term "polychemotherapy" as used herein, is meant to refer to a chemotherapy by several agents. These agents may be administered sequentially, concomitantly, in one or several doses.

Furthermore, reference is made to the following exemplary sequences wherein the SEQ ID NO:s denote the following:

SEQ ID NO: 1 and 2: Two exemplary forms of the PITX2 gene which can also be found under http://www.ncbi.nlm.nih.gov/nuccore/161086966 or NG 007120.1 or http://www.ncbi.nlm.nih.gov/nucleotide?cmd=Retrieve&dopt=GenBank&list uids=13183092 GenBank: AF238048.1

SEQ ID NO:3: An embodiment of a partial PITX2 sequence that has been bisulfite-treated and that has a fully methylated status to which probes for methylated sequences will bind; this is one of the specifically preferred sequences in accordance with embodiments of the present invention.

SEQ ID NO:4: An embodiment of a partial PITX2 sequence that has been bisulfite treated and that is unmethylated; a probe for unmethylated sequences will bind to this SEQ ID NO:4.

SEQ ID NO:5: This is the genomic bisulfite-untreated sequence of SEQ ID NO: 3 and 4.

SEQ ID NO:6: This is a PITX2-specific reverse primer for amplification of a partial sequence of the PITX2-gene, in this case of SEQ ID NO: 3 and 4.

SEQ ID NO:7: This is the forward primer for amplification of SEQ ID NO: 3 and 4.

SEQ ID NO:8: This is a PITX2-specific probe for methylated PITX2.

SEQ ID NO:9: This is a PITX2-specific probe for unmethylated PITX2.

Furthermore, reference is made to the tables, wherein Table 1 shows the adjuvant chemotherapy regimens employed in the patient collective used in the Examples

In the tables, the letters mean the following:

C: Cyclophosphamide
M: Methotrexate
F: 5-Fluoro-Uracile
E: Epirubicine
Example: CMF: Polychemotherapy containing Cyclophosphamide, Methotrexate, 5-FU + sequential Chemotherapy with consecutive poly-chemotherapy regimens.

**Table 2** shows the patients' clinical and histomorphological characteristics. TNBC means triple-negative breast cancer; ABCS means anthracycline-based chemotherapy subgroup; NABCS means non-anthracycline based chemotherapy subgroup; NCS means no chemotherapy subgroup.

**Table 3** shows results of the uni- and multivariable Cox regression analyses of the ABCS-group, i.e. the anthracycline-based chemotherapy subgroup.

**Table 4** shows the specifications concerning the PITX2 probe and primer system. Primer and probe sequences:

| | |
|---|---|
| PITX2-R | ttctaatcctcctttccacaataa (SEQ ID NO:6) |
| PITX2-F | gtaggggagggaagtagatgtt (SEQ ID NO:7) |
| PITX2-Pm | agtcggagtcgggagagcga (containing 3 CpGs) (SEQ ID NO:8) |
| PITX2-Pu | agttggagttgggagagtgaaaggaga (containing 3 CpGs) (SEQ ID NO:9) |

**Table 5** shows the layout plan of preliminary test data for qPCR robustness assessment.

**Table 6** shows the statistical results of a dilution series of MCF-7, tissue X and tissue Y and the corresponding mean values and coefficients of variation. Tissue X and tissue Y are just randomly selected cancer tissues from two patients, from which DNA was isolated, bisulfite-converted and then separately assessed by qPCR in standard dilution series of these two samples.

**Table 7** shows the statistical results of a positive control, negative control, cell line MCF-7 and cell line MDA-MB 231 and the corresponding mean values and coefficients of variation.

**Table 8** shows the findings of various previous studies which examined the methylation of PITX2 in non-TNBC patients.

Furthermore, reference is made to the figures, wherein

**Figure 1** shows the Kaplan-Meier analyses of primary end points using a PITX2 DNA methylation cut-off value of 6.35. More specifically, **Figure 1a** shows the disease-free survival (DFS) by PITX2 DNA methylation; **Figure 1b** shows the metastasis-free survival (MFS) by PITX2 DNA methylation, and **Figure 1c** shows the overall survival (OS) by PITX2 DNA. According to **Figure 1,** the anthracycline-based chemotherapy subgroup (ABCS) was subdivided by the cut-off value of 6.35 into a subgroup with low PITX2 methylation ($\leq 6.35$, n = 18) and into a subgroup with high PITX2 DNA methylation (> 6.35, n = 38). Patients with low methylated PITX2-gene had a significantly worse DFS (p < 0,001, see **Figure 1a**), worse MFS (p = 0.006, see Figure 1b) and worse OS (p = 0,005, see Figure 1c) than patients with high methylated PITX2.

**Figure 2a-c** shows the assessment of qPCR robustness of the PITX2 marker assay. More specifically, for these experiments, two different PITX2 DNA methylation measurements were carried out per each sample. On the x-axis, the hatched column depicts the first measurement/qPCR run and the solid column depicts the second measurement/qPCR run. The determined PIXT2 DNA methylation scores are depicted on the y-axis.

**Figure 3** shows the linear regression analysis for independent assay replicates of TNBC samples. More specifically, for each of the 10 samples, two DNA methylation measurements were carried out. The x-axis depicts the PITX2 DNA methylation score of the first qPCR run and the y-axis the PITX2 DNA methylation score of the second qPCR run. Linear regression analysis resulted in an R-value of 0.93.

**Figure 4a-c** shows a stepwise Cox regression analysis for multivariable biomarker analysis in the anthracycline-based chemotherapy subgroup (ABCS). More specifically, on the y-axis, the different combinations of the variables are pictured with M = PITX2 DNA methylation subgroup ($\leq 6.35$ vs. > 6.35), A = age, G = grading (G1/2 vs. G3), T = tumor size ($\leq 2$ vs. > 2 cm) and N = nodal status (positive vs. negative). The x-axis depicts the different calculated hazard ratios and the respective 95 % confidence intervals.

Furthermore, reference is made to the examples which are given to illustrate, not to limit the present invention.

## Examples

### Example 1

Patient Collective

The inclusion criteria for the current retrospective study were patients with histologically confirmed invasive triple-negative breast cancer, with no signs of distant metastasis at the time of diagnosis, availability of sediment pellets after protein extraction from tumour tissues (as a source for DNA; for methodology see Ref 31), availability of follow-up data and appropriate written informed patient consent. Sediment pellets of 95 patients meeting these predefined criteria were obtained from the Tumour Bank and Mamma CA Database of the Klinikum rechts der Isar, stored at the Department of Obstetrics and Gynecology of the Technical University of Munich. All 95 patients were treated at the Klinikum rechts der Isar, Munich, Germany, between 1991 and 2006. Study approval was obtained from the Ethics Committee of the Medical Faculty of the Technical University of Munich, Germany. Median age of the patients at time of diagnosis was 59 months (range: 27 - 96 months) and the median follow-up time 79 months (range 8 - 216 months).

The inventors aimed for a minimum follow-up period of 60 months for those patients who did not suffer from any events. For five patients this was not achieved since they did not want to participate in any further follow-up examinations. All 95 patients underwent surgical treatment (65 patients: breast conserving therapy, 30 patients: mastectomy). 77 patients received radiotherapy. 23 patients did not receive any chemotherapy, 72 patients were treated with adjuvant chemotherapy. In the majority of cases (n=56) and anthracycline-containing chemotherapy regimen was applied **(Table 1).** Histomorphological and clinical characteristics are depicted in **Table 2.**

Cell Lines and Fresh-Frozen Tissues

Two fresh-frozen breast cancer tissues obtained from the Tumour Bank of the Department of Obstetrics and Gynecology (Klinikum rechts der Isar, Munich, Germany). The breast cancer cell lines MCF-7 and MDA-MB-231 (CLS Cell Lines Service GmbH, Eppelheim, Germany) were employed for preliminary testing and quality assessment.

DNA Extraction

Unless otherwise stated, all the reagents, protocol sheets and materials applied in this study were obtained from Qiagen (Hilden, Germany). Fresh-frozen breast cancer specimens were obtained from the Tumour Bank of the Department of Obstetrics and Gynecology (Klinikum rechts der Isar, Munich, Germany)and processed accordingly.[31] DNA extraction from sediment pellets was performed following the QiAamp DNA Mini and Blood Mini Handbook protocol. Lysis was performed manually, followed by semi-automated extraction with the QIAcube system. Approximately30 mg of sediment pellet was used. The extracted DNA was stored at -20 °C.

DNA Concentration Determination and Adjustment

The Nano Drop 2000c spectrophotometer with appropriate software (Nanodrop / Thermo Fisher Scientific, Wilmington, USA) was used for the determination of the DNA concentration. For PITX2 methylation score measurement optimisation, For each sample, 310 ng of DNA was used in the subsequent bisulfite conversion step. For 13 samples, a DNA concentration step with the Speed Vac System Concentrator 5301 (Eppendorf, Hamburg, Germany) had to be performed.

Bisulfite Conversion

Bisulfite conversion was performed according to the EpiTect Bisulfite Handbook, employing the ABI PCR Cycler (Applied Biosystems, Darmstadt, Germany) (Program details: 1$^{st}$ 5 min at 99 °C, 2$^{nd}$ 25 min at 60 °C, 3$^{rd}$ 5 min at 99 °C, 4$^{th}$ 85 min at 60 °C, 5$^{th}$ 5 min at 99 °C, 6$^{th}$ 175 min at 60 °C). Clean-up of the bisulfite converted DNA was carried out in a semi-automated procedure according to the EpiTect Bisulfite Kit protocol sheet

Quantitative Real Time PCR

PITX2 primers and probes for the methylated and unmethylated DNA status in a duplex system, provided by Qiagen were used (Table 4). qPCR was carried out according to the provider protocol (EpiTect MethyLight Assay Hs_PITX2) using an ABI 7000 Taqman System (Applied Biosystems, Darmstadt, Germany) (Run details: 1$^{st}$ 15 min at 95 °C; 2$^{nd}$ 48 cycles comprising of 15 sec at 95 °C and 1 min at 60 °C) and the Quantitect 2x QPCR Mastermix (Qiagen, Hilden, Germany) in a final volume of 20 $\mu$l. Each specimen was measured in triplicates. A minimum of two runs per specimen was performed. On each plate, a positive control comprising of fully methylated bisulfite-converted human control DNA, a negative control (RNAse-free water) and 7.75 ng of bisulfite-converted MCF-7 DNA were included.

Statistics and Quality Assessment

Reporting of this study was carried out observing the REMARK criteria.[22] For calculation of PITX2 DNA methylation scores, a modified 2exp-$\Delta\Delta$CT-method was used as described earlier (Ref 11). CT mean values of triplicates were calculated for the methylated and unmethylated state, which were used for calculation of the final methylation scores. For quality reasons, results with both CT values (methylated and unmethylated) greater than 38 cycles were excluded, in this case the measurements had to be repeated. Mean values, standard deviations and coefficients of variation of the different qPCR runs were calculated. In case the coefficient of variation exceeded 0.3, a third qPCR run was carried out and the results calculated accordingly. Primary endpoints were defined as overall survival (OS), metastasis-free survival (MFS; time to detection of distant metastasis) and disease-free survival (DFS; period after primary disease elimination in which no disease was detected). The date of primary surgery was considered as the follow-up index date.

In order to discriminate low- and high-risk patients with regards to DFS, MFS and OS, optimised cut-off values were calculated with the "maximum-selected log-rank statistic" using the maxstat.test function as implemented from the program library "maxstat" of the program "R" (R Development Core Team 2012).[14, 35] Kaplan-Meier analyses were carried out to estimate and display empirical survivor functions for OS, MFS and DFS. The Logrank test was used for calculating the respective p-values. Cox regression models were used for univariate estimation of hazard ratios (HRs) with regards to DFS, MFS and OS. Due to the limited event numbers, multivariable analysis was carried out in an exploratory way. Furthermore, covariates were added stepwise to the variable PITX2 methylation scores and the according hazard rations (HR) and 95 % confidence intervals were depicted in Forest plot diagrams in order to test whether PITX2 methylation adds independent additional information to the other factors.

## Example 2

Detailed Results of Preliminary Tests

In order to evaluate the impact of the different processing steps (DNA extraction, bisulfite conversion and qPCR run), a preliminary test consisting of three different levels was accomplished **(Table 5 and Fig. 2+3).** Ten samples of the triple-negative patient collective were chosen randomly. Whether diverse DNA methylation results derive from the same bisulfite converted DNA measured in two separate qPCR runs was examined on the first level. For three specimens, the substrates which were applied to the two distinct qPCR runs derived from the same DNA extraction and bisulfite conversion, leaving the qPCR run the only variable factor **(Fig. 2a).** For each sample, the coefficient of variation regarding the DNA methylation scores measured in two different qPCR runs was calculated. The mean value of these coefficients of variation was 0.09. On the second level, the impact of bisulfite conversion was examined using three other samples. This time, the substrate of one sample derived from the same DNA extraction but both bisulfite conversion and qPCR constituted differing factors which might possibly influence the score results (Fig. 2b). The mean value of the coefficients of variation of the measured methylation scores was determined 0.07. Finally, four different specimens underwent an experiment set-up in which DNA extraction, bisulfite conversion as well as qPCR runs were different for the two reagents measured per each sample. 0.14 was the calculated mean value of coefficients of variation **(Fig. 2c).** When plotting the results of the two different runs per each of the ten samples, a high correlation (R >0.93) between the two PITX2 DNA methylation scores was observed with a mean value of coefficient of variation of 0.10 **(Fig. 3).**

Score reproducibility was demonstrated through gradual and serial dilution series and subsequent qPCR runs using 7.75 ng of bisulfite-converted DNA extracted from the MCF-7 cell line and two fresh-frozen breast cancer tissues (Tissue X and Y; **Table 6**). By serial dilutions, the applied bisulfite converted DNA concentration was bisected with each dilution step (6 steps were carried out resulting in concentrations of 100 / 50 / 25 / 12.5 / 6.25 / 3.125 / 1.5625 %), whereas through gradual dilution the original concentration was reduced by 20 % with each of the 4 steps (leading to concentrations of 100 / 80 / 60 / 40 / 20 %). For the MCF-7 cell line, one serial and two gradual dilutions were accomplished, whereas the fresh-frozen breast cancer tissues each underwent one gradual dilution. The mean value and coefficient of variation of the different dilution steps was calculated for each dilution series ranging between 0.00 and 0.06 **(Table 6).**

For quality assurance of the different qPCR runs, positive controls, negative controls and the MCF-7 cell line were included in each run, whereas another control cell line with median PITX2 DNA methylation levels, MDA-MB-231, was only included in 27.3 % of all runs. For the PITX2 DNA methylation scores of the different runs and controls, the respective mean values and coefficients of variation were calculated **(Table 7).** Stable scores for the controls were obtained throughout the different qPCR runs.

## Example 3

Preliminary Tests

Preliminary tests were carried out in order to assess the assay stability **(Fig, 2-3 and Tables 5-7).** Score reproducibility was demonstrated through gradual and serial dilution series and subsequent qPCR runs using 7.75 ng of bisulfite-converted DNA extracted from MCF-7 cells and two fresh-frozen breast cancer tissues (Tissue X and Y; **Table 6**). The calculated low coefficients of variation (highest 0.06; **Table 6**) indicate that even with low concentrations of bisulfite-converted DNA, PITX2 methylation scores can be determined reliably. For further quality assurance of the different qPCR runs, positive controls, negative controls and the MCF-7 cell line were included in each run. The according low coefficients of variation (highest 0.08; **Table 7**) indicate that stable scores could be obtained throughout the different qPCR runs. Comparison of methylation scores of ten randomly chosen triple-negative samples, which were processed in different turns **(Table 5** and **Fig. 2/3),** revealed a low median coefficient of variation of 0.10 and high correlation (R > 0.93; *Fig.* 3).

PITX2 DNA Methylation Scores, Clinical Outcome and Subgroup Definition

Valid PITX2 DNA methylation scores were obtained for all patients. The median methylation score was 10.05. Approximately 75.3 % of the patients survived 5 years; the estimated 5-year DFS probability was 74.6 % and the estimated 5-year MFS probability 80.3 %. The majority of events occurred within 5 years (92.0 % of disease recurrences, 90.0 % of metastases and 92.0 % of deaths). To analyse whether PITX2 methylation might constitute a prognostic or predictive marker in TNBC, subgroup analyses were performed. According to the adjuvant treatment, three different subgroups were defined: no-chemotherapy subgroup (NCS), non-anthracycline-based chemotherapy subgroup (NABCS) and anthracycline-based chemotherapy subgroup (ABCS). For detailed clinical and histomorphological characteristics see **Table 2.**

Cut-Off Definition and Application

Due to the fact that an application of the PITX2 DNA methylation cut-off value defined by *Harbeck et al* of 22.9 [11] did not lead to any significant risk group separation, the 'maxstat.test' R-function was used in order to search for optimised cut-off values. Using this conservative test, which already accounts for the issue of multiple testing, no statistical significant cut-off value was found when analysing the whole TNBC collective (DFS: cut-off 6.35, p = 0.529; MFS cut-off 6.35, p > 0.99; OS cut-off 7.34, p = 0.611). As therapy based subgroup analyses were of special interest to the inventors, the search for optimised cut-off values was also performed in the ABCS, NABCS and NCS. In the anthracycline-based chemotherapy subgroup (ABCS) a cut-off value of 6.35 was calculated for the three primary endpoints. Only for DFS the risk group separation within the anthracycline-based chemotherapy subgroup was found to be statistically significant (DFS p = 0.015; OS p = 0.091; MFS p = 0.275). Neither for the NABCS nor the NCS, statistical significant cut-off values could be defined.

In the ABCS, Kaplan Meier analyses were carried out using the defined cut-off value of 6.35 for the endpoints DFS, MFS and OS in order to estimate and display empirical survival tendencies. Low methylation was associated with statistically significant worse prognosis regarding DFS (p < 0.001, 5-year DFS 35.6 % vs. 83.5 %,**Fig. 1a**), MFS (p = 0.006, 5-year DFS 53.3 % vs 86.5 %, **Fig. 1b**) and OS (p = 0.005, 5-year DFS 50.0 % vs 80.9 %, Fig. 1c).

ABCS - Uni- and Multivariable Cox Regression Analysis

In order to evaluate the impact of PITX2 DNA methylation scores on clinical outcome in the ABCS, uni- and multivariable Cox regression analyses were carried out for the different endpoints. Due to the limited events numbers, multivariable analysis was only carried out with exploratory intentions. Therefore, stepwise inclusion of the established clinical/histomorphological factors age, tumor grading (G3 vs. G1/2), tumour size (> 2 vs. ≤ 2cm) and nodal status (positive vs. negative) as covariates was necessary when testing whether PITX2 DNA methylation (≤6.35 vs. > 6.35) constitutes a stable and independent variable. The calculated hazard ratios of PITX2 DNA methylation obtained from stepwise addition of covariates for the three endpoints were stable and with regards to DFS each of them was statistically significant (Fig 4a). However, two 95 % confidence intervals exceeded the value of 1.0 when analysing MFS and one when analysing OS, leading to statistical insignificance **(Fig. 4b and 4c).** Both in univariate (DFS: HR = 5.36, 95 % CI 2.06 - 13.95; MFS: HR = 3.95, 95 % CI 1.36 - 11.46; OS: HR = 3.78, 95 % CI 1.40 - 10.20) and multivariable (DFS: HR = 6.40, 95 % CI 1.96 - 20.88; MFS: HR = 3.89, 95 % CI 1.09 - 13.95; OS: HR = 3.62, 95 % CI 1.03 - 12.72) Cox regression, PITX2 DNA methylation was found to contribute significant additional information with regards to the three endpoints **(Table 3).** Out of the established factors, only age contributed significant information in univariate analysis of OS (HR = 1.05, 95 % CI 1.01 - 1.10).

Preliminary Tests and Quality Assessment

To the inventors' knowledge, the current study is the first to analyse PITX2 DNA methylation of TNBC DNA obtained from sediment pellets. Other research groups who examined this marker in non-TNBC used e.g. FFPE tissue sections or fresh-frozen tumour specimens as primary material.[11, 12, 21, 25] On this basis, preliminary tests had to be carried out in order to prove applicability of sediment pellets for measuring DNA methylation markers and to show that the achieved PITX2 scores are reliable and reproducible. High standards of quality criteria were applied. In accordance with the criteria used in the multicenter study of Harbeck *et al*.[11], measurements with both methylated and unmethylated CT values greater than 38 were excluded. Additionally, the quality criteria was extended since for each sample the coefficient of variation of two different qPCR results was calculated and in case of it exceeding 0.3 another qPCR run was carried out. The impact of sample processing techniques on obtained PITX2 DNA methylation scores was examined (*Example 2 above).* Low mean values of coefficient of variation (0.09, 0.07 and 0.14 respectively) and a high correlation between PITX2 DNA methylation scores using material from the same samples processed in different turns were revealed (r > 0.93). This indicated that the influence of processing techniques on PITX2 DNA methylation scores can be neglected. Stable measurements gained from dilution series showed that even when applying small amounts of bisulfite converted DNA, robust results can be achieved with the used PITX2 primer and probe system. These results indicate a reliable workflow and provide evidence for the use of PITX2 DNA methylation score obtained from sediment pellets as a robust marker.

Clinical Outcome, Clinical Data and PITX2 DNA Methylation Score

The estimated 5-year OS rate (75.3 %) was similar to the 5-year OS rate of the triple-negative cohort analysed by Bauer et al (77 %).[5] In concordance with the reported bad prognosis [23, 34, 36] of triple-negative diseases, the relatively low 5-year MFS (80.3 %) and DFS rates (74.6 %) in the current collective were not surprising.

The tendency of TNBC towards early onset[1], higher grade[2] and larger size[7, 18, 37] was obvious in the current collective as the median age at time of diagnosis was 59, 82.1 % of examined tumours were of grade 3 and 65.3 % were found to be larger than 2.0 cm in diameter. With 45.2 % of patients being nodal positive, lymph node involvement was relatively common.

The median PITX2 methylation score of the analysed TNBC was 10.05, considerably lower than in non-TNBC.[11, 21, 25]. In different types of tumours, PITX2 expression levels seem to have different consequences and aberrant methylation levels were found in various malignant cancers. Although no study revealed a direct association between PITX2 hypomethylation and tumourigenesis, PITX2 over-expression was found in several cancer types such as non-functional pituitary adenomas[24] and ovarian cancer.[8] On the other hand, PITX2 downregulation and hypermethylation was present in e.g. colorectal[13] and prostate tumours.[3] These results indicate that PITX2 might possess a dose-dependent oncogenic potential in different tissues and that one of the factors disturbing its physiological balance might be epigenetic deregulation via DNA methylation.

PITX2 DNA methylation as predictive marker in TNBC

To the inventors' knowledge, this is the first study examining whether PITX2 DNA methylation can serve as a predictive marker in triple-negative breast cancer. As already mentioned, several previous studies examined PITX2 DNA methylation in non-TNBC and found that high DNA methylation scores were associated with poor clinical outcome (Table 8). In a study by Hartmann *et al* on estrogen receptor-positive, nodal-positive breast cancer patients who had received an adjuvant anthracycline-based chemotherapy, high PITX2 DNA methylation was associated with poor DFS, MFS and OS.[12] Contrary to its role in non-TNBC, low PITX2 methylation seems to be a statistically independent marker, predicting response to anthracyclines in a TNBC collective whereas no association between PITX2 DNA methylation and clinical outcome in the NABCS and NCS was found.

In the ABCS, low PITX2 DNA methylation was associated with poor DFS, MFS and OS. Uni- and multivariable Cox analysis as well as stepwise inclusion of covariates suggested PITX2 DNA methylation as a statistically independent and stable factor. Advanced age was found to be the only established prognostic factor which was marginally associated with poor OS in univariate Cox analysis.

The current results, which indicate a reverse relationship between PITX2 DNA methylation and outcome in anthracycline-receiving TNBC compared to non-TNBC, are surprising. However, they lend support to PITX2 DNA methylation being a highly valuable marker as it can influence therapeutic decisions in triple-negative breast cancer. In TNBC, PITX2 DNA methylation is useful in order to identify patients who derive sufficient treatment through anthracyclines. Furthermore, the resistant subgroup with low methylation scores might need more intensified therapy or simply different treatment (e.g. PARP inhibitors, platinum-based chemotherapies or CMF) and thus should be spared the negative side-effects of anthracyclines.

*Table 1: Adjuvant Chemotherapy Regimens*

| Regimen | No. of Patients (N = 72) | % |
|---|---|---|
| CMF | 16 | 22.2 |
| FEC | 21 | 29.2 |
| EC | 12 | 16.7 |
| EC + CMF | 3 | 4.2 |
| Anthracycline- plusTaxane-Containing Polychemotherapy | 9 | 12.5 |
| Other Anthracycline-cContaining Polychemotherapy | 11 | 15.3 |

*Table 2: Patients' Clinical and Histomorphological Characteristics*

| | TNBC | | ABCS | | NABCS | | NCS | |
|---|---|---|---|---|---|---|---|---|
| Characteristic | No. of Patients (N = 95) | % | No. of Patients (N = 56) | % | No. of Patients (N = 16) | % | No. of Patients (N = 23) | % |
| **Tumour Grade** | | | | | | | | |
| 1 | 6 | 6.3 | - | - | - | - | 6 | 26.1 |
| 2 | 9 | 9.5 | 4 | 7.1 | 2 | 12.5 | 3 | 13.0 |
| 3 | 78 | 82.1 | 50 | 89.3 | 14 | 87.5 | 14 | 60.9 |
| No Data | 2 | 2.1 | 2 | 3.6 | - | - | - | - |
| **Nodal Status** | | | | | | | | |
| pN0 | 50 | 52.6 | 27 | 48.2 | 9 | 56.3 | 14 | 60.9 |
| pN1 | 33 | 34.7 | 20 | 35.7 | 7 | 43.8 | 6 | 26.1 |
| pN2 | 6 | 6.3 | 6 | 10.7 | - | - | - | - |
| pN3 | 4 | 4.2 | 2 | 3.6 | - | - | 2 | 8.7 |
| No Data | 2 | 2.1 | 1 | 1.8 | - | - | 1 | 4.3 |
| **Tumour Size (cm)** | | | | | | | | |
| ≤ 2 | 29 | 30.5 | 18 | 32.1 | 6 | 37.5 | 5 | 21.7 |
| > 2 | 62 | 65.3 | 36 | 64.3 | 9 | 56.3 | 17 | 73.9 |
| No Data | 4 | 4.2 | 2 | 3.6 | 1 | 6.3 | 1 | 4.3 |
| **Age at Time of Diagnosis, Years** | | | | | | | | |
| < 50 | 67 | 70.5 | 20 | 35.7 | 4 | 25.0 | 4 | 17.4 |
| ≥ 50 | 28 | 29.5 | 36 | 64.3 | 12 | 75.0 | 19 | 82.6 |
| **Histological Subtype** | | | | | | | | |
| Invasive Ductal | 66 | 69.5 | 42 | 75.0 | 13 | 81.3 | 11 | 47.8 |
| Medullary | 9 | 9.5 | 7 | 12.5 | 1 | 6.3 | 1 | 4.3 |
| Lobular | 4 | 4.2 | 1 | 1.8 | 1 | 6.3 | 2 | 8.7 |
| Tubular | 2 | 2.1 | - | - | - | - | 2 | 8.7 |
| Others | 13 | 13.7 | 6 | 10.7 | 1 | 6.3 | 6 | 26.1 |
| No Data | 1 | 1.1 | - | - | - | - | 1 | 4.3 |
| **Disease Recurrence** | | | | | | | | |
| Yes | 25 | 26.3 | 18 | 32.1 | 1 | 6.3 | 6 | 26.1 |
| Median Time to Disease Recurrence | 27 Months (Range 4-101) | | 24.5 Months (Range 4-72) | | 51 Months | | 31.5 Months (Range 9-101) | |
| No | 70 | 73.7 | 38 | 67.9 | 15 | 93.8 | 17 | 73.9 |
| **Deceased** | | | | | | | | |
| Yes | 25 | 26.3 | 16 | 28.6 | 1 | 6.3 | 8 | 34.8 |
| Median Time to Death | 31 Months (Range 8-70) | | 25 Months (Range 8-60) | | 55 Months | | 43.5 Months (Range 8-70) | |
| No | 70 | 73.7 | 40 | 71.4 | 15 | 93.8 | 15 | 65.2 |
| **Metastasised** | | | | | | | | |
| Yes | 20 | 21.1 | 14 | 25.0 | 1 | 6.3 | 5 | 21.7 |
| Median Time to Metastasis | 25.5 Months (Range 4-101) | | 19.5 Months (Range 4-71) | | 51 Months | | 39 Months (Range 24-101) | |
| No | 75 | 78.9 | 42 | 75.0 | 15 | 93.8 | 18 | 78.3 |

*Table 3: ABCS Uni- and Multivariable Cox Regression Analyses*

| Variable | Hazard ratio | 95 % CI | P | N |
|---|---|---|---|---|
| **DFS** – Univariate Analysis | | | | |
| Age | 1.02 | 0.98 – 1.06 | 0.415 | 56 |
| Tumour Grading | 1.30 | 0.17 – 9.83 | 0.798 | 54 |
| Tumour Size | 2.75 | 0.80 – 9.52 | 0.109 | 54 |
| Nodal Status | 1.55 | 0.60 – 4.00 | 0.365 | 55 |
| PITX2 DNA Methylation | 5.36 | 2.06 – 13.95 | **0.001** | 56 |
| **DFS** – MultivariableAnalysis | | | | |
| Age | 1.00 | 0.96 – 1.05 | 0.981 | 51 |
| Tumour Grading | 0.86 | 0.10 – 7.78 | 0.893 | 51 |
| Tumour Size | 1.13 | 0.26 – 5.01 | 0.871 | 51 |
| Nodal Status | 2.04 | 0.61 – 6.84 | 0.249 | 51 |
| PITX2 DNA Methylation | 6.40 | 1.96 – 20.88 | **0.002** | 51 |
| **MFS** – Univariate Analysis | | | | |
| Age | 1.03 | 0.98 – 1.08 | 0.209 | 56 |
| Tumour Grading | 0.94 | 0.12 – 7.24 | 0.952 | 54 |
| Tumour Size | 3.25 | 0.73 – 14.53 | 0.123 | 54 |
| Nodal status | 1.78 | 0.60 – 5.31 | 0.301 | 55 |
| PITX2 DNA Methylation | 3.95 | 1.36 – 11.46 | **0.011** | 56 |
| **MFS** – Multivariable Analysis | | | | |
| Age | 1.02 | 0.96 – 1.07 | 0.546 | 51 |
| Tumour Grading | 0.52 | 0.05 – 5.15 | 0.577 | 51 |
| Tumour Size | 1.78 | 0.29 – 10.88 | 0.531 | 51 |
| Nodal Status | 1.61 | 0.44 – 5.88 | 0.471 | 51 |
| PITX2 DNA Methylation | 3.89 | 1.09 – 13.95 | **0.037** | 51 |
| **OS** – Univariate Analysis | | | | |
| Age | 1.05 | 1.01 – 1.10 | **0.032** | 56 |
| Tumour Grading | 1.09 | 0.14 – 8.30 | 0.933 | 54 |
| Tumour Size | 1.62 | 0.52 – 5.02 | 0.404 | 54 |
| Nodal Status | 1.46 | 0.52 – 4.11 | 0.471 | 55 |
| PITX2 DNA Methylation | 3.78 | 1.40 – 10.20 | **0.009** | 56 |
| **OS** –Multivariable Analysis | | | | |
| Age | 1.05 | 0.99 – 1.11 | 0.092 | 56 |
| Tumour Grading | 0.54 | 0.06 – 5.23 | 0.594 | 56 |
| Tumour Size | 1.12 | 0.23 – 5.58 | 0.886 | 56 |
| Nodal Status | 0.99 | 0.30 – 3.21 | 0.982 | 56 |
| PITX2 DNA Methylation | 3.62 | 1.03 – 12.72 | **0.045** | 56 |

*Table 4: Specifications Regarding PITX2 Probe and Primer System (According to Provider)*

| | |
|---|---|
| Entrez gene ID | 5308 |
| Amplicon length | 144 |
| Reference sequence (Ref Seq) ID | NT_016354.18 |
| Detected CpG in Ref Seq | 3 CpG in 36106573 - 36106600 |

*Table 5: Layout Plan of Preliminary Test data for qPCR robustness assessment*

| Level | Comparison of | No. of samples | DNA extraction | Bisulfite conversion | qPCR run |
|---|---|---|---|---|---|
| 1 | 2 Different qPCR Runs | 3 | Same | Same | Different |
| 2 | 2 Different Bisulfite Conversions | 3 | Same | Different | Different |
| 3 | 2 Different DNA Extractions | 4 | Different | Different | Different |

*Table 6: Dilution Series of MCF-7, Tissue X and Tissue Y and According Mean Values & Coefficients of Variation*

| Dilution Series of | Type of Dilution | No. of Replicates per Dilution Step | Mean value | Coefficient of Variation |
|---|---|---|---|---|
| MCF-7 | Serial | 3 | 96.9 | 0.00 |
| MCF-7 | Gradual | 3 | 92.9 | 0.01 |
| MCF-7 | Gradual | 3 | 95.7 | 0.00 |
| Tissue X | Gradual | 3 | 66.9 | 0.04 |
| Tissue Y | Gradual | 3 | 44.6 | 0.06 |

*Table 7: Positive Control, Negative Control, MCF-7 and MDA-MB-231 and According Mean Values & Coefficients of Variation*

| Control | No. of Replicates per qPCR Run | No. of Experiments | Mean Value | Coefficient of Variation |
|---|---|---|---|---|
| Positive Control | 3 | 22 | 92.3 | 0.01 |
| Negative Control | 2 | 22 | | - |
| MCF-7 | 3 | 22 | 94.7 | 0.01 |
| MDA-MB-231 | 3 | 6 | 70.4 | 0.08 |

*Table 8: Association Between PITX2 Methylation and Outcome in Non-TNBC*

| Research Group | Patient Collective | High PITX2 Methylation Associated with |
|---|---|---|
| Maier *et al* [21] | HR+, N-, Adjuvant Tamoxifen Monotherapy | Poor MFS |
| Nimmrich *et al* [25] | HR+, N-, Untreated | Poor MFS and OS |
| Harbeck *et al* [11] | ER and/or PR+, N-, Adjuvant Tamoxifen Monotherapy | Poor MFS |
| Hartmann *et al* [12] | ER+, N+, Adjuvant Anthracycline-based Polychemotherapy | Poor MFS, DFS, OS |


1. Krebs in Deutschland 2007/2008. 8. Ausgabe. Robert Koch-Institut (Hrsg) und die Gesellschaft der epidemiologischen Krebsregister in Deutschland e.V. (Hrsg). Berlin, 2012.

2. Albergaria A, Ricardo S, Milanezi F, Carneiro V, Amendoeira I, Vieira D, Cameselle-Teijeiro J, Schmitt F (2011) Nottingham Prognostic Index in triple-negative breast cancer: a reliable prognostic tool? BMC cancer 11:299. doi: 10.1186/1471-2407-11-299

3. Banez LL, Sun L, van Leenders GJ, Wheeler TM, Bangma CH, Freedland SJ, Ittmann MM, Lark AL, Madden JF, Hartman A, Weiss G, Castanos-Velez E (2010) Multicenter clinical validation of PITX2 methylation as a prostate specific antigen recurrence predictor in patients with post-radical prostatectomy prostate cancer. The Journal of urology 184:149-156. doi: 10.1016/j.juro.2010.03.012

4. Basu M, Roy SS (2013) Wnt/beta-catenin pathway is regulated by PITX2 homeodomain protein and thus contributes to the proliferation of human ovarian adenocarcinoma cell, SKOV-3. The Journal of biological chemistry 288:4355-4367. doi: 10.1074/jbc.M112.409102

5. Bauer KR, Brown M, Cress RD, Parise CA, Caggiano V (2007) Descriptive analysis of estrogen receptor (ER)-negative, progesterone receptor (PR)-negative, and HER2-negative invasive breast cancer, the so-called triple-negative phenotype: a population-based study from the California cancer Registry. Cancer 109:1721-1728. doi: 10.1002/cncr.22618

6. Dent R, Trudeau M, Pritchard KI, Hanna WM, Kahn HK, Sawka CA, Lickley LA, Rawlinson E, Sun P, Narod SA (2007) Triple-negative breast cancer: clinical features and patterns of recurrence. Clin Cancer Res 13:4429-4434. doi: 10.1158/1078-0432.CCR-06-3045

7. Elias AD (2010) Triple-negative breast cancer: a short review. American journal of clinical oncology 33:637-645. doi: 10.1097/COC.0b013e3181b8afcf

8. Fung FK, Chan DW, Liu VW, Leung TH, Cheung AN, Ngan HY (2012) Increased expression of PITX2 transcription factor contributes to ovarian cancer progression. PloS one 7:e37076. doi: 10.1371/journal.pone.0037076

9. Geyer FC, Lacroix-Triki M, Savage K, Arnedos M, Lambros MB, MacKay A, Natrajan R, Reis-Filho JS (2011) beta-Catenin pathway activation in breast cancer is associated with triple-negative phenotype but not with CTNNB1 mutation. Modern pathology : an official journal of the United States and Canadian Academy of Pathology, Inc 24:209-231. doi: 10.1038/modpathol.2010.205

10. Hammond ME, Hayes DF, Dowsett M, Allred DC, Hagerty KL, Badve S, Fitzgibbons PL, Francis G, Goldstein NS, Hayes M, Hicks DG, Lester S, Love R, Mangu PB, McShane L, Miller K, Osborne CK, Paik S, Perlmutter J, Rhodes A, Sasano H, Schwartz JN, Sweep FC, Taube S, Torlakovic EE, Valenstein P, Viale G, Visscher D, Wheeler T, Williams RB, Wittliff JL, Wolff AC, American Society of Clinical O, College of American P (2010) American Society of Clinical Oncology/College of American Pathologists guideline recommendations for immunohistochemical testing of estrogen and progesterone receptors in breast cancer (unabridged version). Archives of pathology & laboratory medicine 134:e48-72. doi: 10.1043/1543-2165-134.7.e48

11. Harbeck N, Nimmrich I, Hartmann A, Ross JS, Cufer T, Grutzmann R, Kristiansen G, Paradiso A, Hartmann O, Margossian A, Martens J, Schwope I, Lukas A, Muller V, Milde-Langosch K, Nahrig J, Foekens J, Maier S, Schmitt M, Lesche R (2008) Multicenter study using paraffin-embedded tumor tissue testing PITX2 DNA methylation as a marker for outcome prediction in tamoxifen-treated, node-negative breast cancer patients. Journal of clinical oncology : official journal of the American Society of Clinical Oncology 26:5036-5042. doi: 10.1200/JCO.2007.14.1697

12. Hartmann O, Spyratos F, Harbeck N, Dietrich D, Fassbender A, Schmitt M, Eppenberger-Castori S, Vuaroqueaux V, Lerebours F, Welzel K, Maier S, Plum A, Niemann S, Foekens JA, Lesche R, Martens JW (2009) DNA methylation markers predict outcome in node-positive, estrogen receptor-positive breast cancer with adjuvant anthracycline-based chemotherapy. Clinical cancer research : an official journal of the American Association for Cancer Research 15:315-323. doi: 10.1158/1078-0432.CCR-08-0166

13. Hirose H, Ishii H, Mimori K, Tanaka F, Takemasa I, Mizushima T, Ikeda M, Yamamoto H, Sekimoto M, Doki Y, Mori M (2011) The significance of PITX2 overexpression in human colorectal cancer. Annals of surgical oncology 18:3005-3012. doi: 10.1245/s 10434-011-1653-z

14. Hothorn T (2011) maxstat: Maximally Selected Rank Statistics. In: R package version 0.7-14.

15. Jovanovic J, Ronneberg JA, Tost J, Kristensen V (2010) The epigenetics of breast cancer. Molecular oncology 4:242-254. doi: 10.1016/j.molonc.2010.04.002

16. Khasraw M, Bell R, Dang C (2012) Epirubicin: is it like doxorubicin in breast cancer? A clinical review. Breast 21:142-149. doi: 10.1016/j.breast.2011.12.012

17. Kioussi C, Briata P, Baek SH, Rose DW, Hamblet NS, Herman T, Ohgi KA, Lin C, Gleiberman A, Wang J, Brault V, Ruiz-Lozano P, Nguyen HD, Kemler R, Glass CK, Wynshaw-Boris A, Rosenfeld MG (2002) Identification of a Wnt/Dvl/beta-Catenin --> Pitx2 pathway mediating cell-type-specific proliferation during development. Cell 111:673-685.

18. Kreike B, van Kouwenhove M, Horlings H, Weigelt B, Peterse H, Bartelink H, van de Vijver MJ (2007) Gene expression profiling and histopathological characterization of triple-negative/basal-like breast carcinomas. Breast cancer research : BCR 9:R65. doi: 10.1186/bcr1771

19. Lee WK, Chakraborty PK, Thevenod F (2013) Pituitary homeobox 2 (PITX2) protects renal cancer cell lines against doxorubicin toxicity by transcriptional activation of the multidrug transporter ABCB1. International journal of cancer. Journal international du cancer 133:556-567. doi: 10.1002/ijc.28060

20. Luu HH, Zhang R, Haydon RC, Rayburn E, Kang Q, Si W, Park JK, Wang H, Peng Y, Jiang W, He TC (2004) Wnt/beta-catenin signaling pathway as a novel cancer drug target. Current cancer drug targets 4:653-671.

21. Maier S, Nimmrich I, Koenig T, Eppenberger-Castori S, Bohlmann I, Paradiso A, Spyratos F, Thomssen C, Mueller V, Nahrig J, Schittulli F, Kates R, Lesche R, Schwope I, Kluth A, Marx A, Martens JW, Foekens JA, Schmitt M, Harbeck N, European Organisation for R, Treatment of Cancer PathoBiology g (2007) DNA-methylation of the homeodomain transcription factor PITX2 reliably predicts risk of distant disease recurrence in tamoxifen-treated, node-negative breast cancer patients-Technical and clinical validation in a multi-centre setting in collaboration with the European Organisation for Research and Treatment of Cancer (EORTC) PathoBiology group. European journal of cancer 43:1679-1686. doi: 10.1016/j.ejca.2007.04.025

22. McShane LM, Altman DG, Sauerbrei W, Taube SE, Gion M, Clark GM, Statistics Subcommittee of the NCIEWGoCD (2005) REporting recommendations for tumor MARKer prognostic studies (REMARK). Nature clinical practice. Oncology 2:416-422.

23. Mersin H, Yildirim E, Berberoglu U, Gulben K (2008) The prognostic importance of triple negative breast carcinoma. Breast 17:341-346. doi: 10.1016/j.breast.2007.11.031

24. Moreno CS, Evans CO, Zhan X, Okor M, Desiderio DM, Oyesiku NM (2005) Novel molecular signaling and classification of human clinically nonfunctional pituitary adenomas identified by gene expression profiling and proteomic analyses. Cancer research 65:10214-10222. doi: 10.1158/0008-5472.CAN-05-0884

25. Nimmrich I, Sieuwerts AM, Meijer-van Gelder ME, Schwope I, Bolt-de Vries J, Harbeck N, Koenig T, Hartmann O, Kluth A, Dietrich D, Magdolen V, Portengen H, Look MP, Klijn JG, Lesche R, Schmitt M, Maier S, Foekens JA, Martens JW (2008) DNA hypermethylation of PITX2 is a marker of poor prognosis in untreated lymph node-negative hormone receptor-positive breast cancer patients. Breast cancer research and treatment 111:429-437. doi: 10.1007/s10549-007-9800-8

26. Novak P, Jensen T, Oshiro MM, Watts GS, Kim CJ, Futscher BW (2008) Agglomerative epigenetic aberrations are a common event in human breast cancer. Cancer research 68:8616-8625. doi: 10.1158/0008-5472.CAN-08-1419

27. Oakman C, Moretti E, Galardi F, Biagioni C, Santarpia L, Biganzoli L, Di Leo A (2011) Adjuvant systemic treatment for individual patients with triple-negative breast cancer. Breast 20 Suppl 3:S135-141. doi: 10.1016/S0960-9776(11)70311-3

28. Perou CM, Sorlie T, Eisen MB, van de Rijn M, Jeffrey SS, Rees CA, Pollack JR, Ross DT, Johnsen H, Akslen LA, Fluge O, Pergamenschikov A, Williams C, Zhu SX, Lonning PE, Borresen-Dale AL, Brown PO, Botstein D (2000) Molecular portraits of human breast tumours. Nature 406:747-752. doi: 10.1038/35021093

29. Prat A, Parker JS, Karginova O, Fan C, Livasy C, Herschkowitz JI, He X, Perou CM (2010) Phenotypic and molecular characterization of the claudin-low intrinsic subtype of breast cancer. Breast cancer research : BCR 12:R68. doi: 10.1186/bcr2635

30. Schatz P, Dietrich D, Koenig T, Burger M, Lukas A, Fuhrmann I, Kristiansen G, Stoehr R, Schuster M, Lesche R, Weiss G, Corman J, Hartmann A (2010) Development of a diagnostic microarray assay to assess the risk of recurrence of prostate cancer based on PITX2 DNA methylation. The Journal of molecular diagnostics : JMD 12:345-353. doi: 10.2353/jmoldx.2010.090088

31. Schmitt M, Mengele K, Schueren E, Sweep FC, Foekens JA, Brunner N, Laabs J, Malik A, Harbeck N, European Organisation for R, Treatment of Cancer Pathobiology G (2007) European Organisation for Research and Treatment of Cancer (EORTC) Pathobiology Group standard operating procedure for the preparation of human tumour tissue extracts suited for the quantitative analysis of tissue-associated biomarkers. European journal of cancer 43:835-844. doi: 10.1016/j.ejca.2007.01.008

32. Shen C, Huang Y, Liu Y, Wang G, Zhao Y, Wang Z, Teng M, Wang Y, Flockhart DA, Skaar TC, Yan P, Nephew KP, Huang TH, Li L (2011) A modulated empirical Bayes model for identifying topological and temporal estrogen receptor alpha regulatory networks in breast cancer. BMC systems biology 5:67. doi: 10.1186/1752-0509-5-67

33. Sorlie T, Perou CM, Tibshirani R, Aas T, Geisler S, Johnsen H, Hastie T, Eisen MB, van de Rijn M, Jeffrey SS, Thorsen T, Quist H, Matese JC, Brown PO, Botstein D, Lonning PE, Borresen-Dale AL (2001) Gene expression patterns of breast carcinomas distinguish tumor subclasses with clinical implications. Proceedings of the National Academy of Sciences of the United States of America 98:10869-10874. doi: 10.1073/pnas.191367098

34. Tan DS, Marchio C, Jones RL, Savage K, Smith IE, Dowsett M, Reis-Filho JS (2008) Triple-negative breast cancer: molecular profiling and prognostic impact in adjuvant anthracycline-treated patients. Breast cancer research and treatment 111:27-44. doi: 10.1007/s 10549-007-9756-8

35. Team RDC (2012) R: A language and environment for statistical computing. In:R Foundation for Statistical Computing, Vienna, Austria

36. Theriault RL, Litton JK, Mittendorf EA, Chen H, Meric-Bernstam F, Chavez-Macgregor M, Morrow PK, Woodward WA, Sahin A, Hortobagyi GN, Gonzalez-Angulo AM (2011) Age and survival estimates in patients who have node-negative T1 ab breast cancer by breast cancer subtype. Clinical breast cancer 11:325-331. doi: 10.1016/j.clbc.2011.05.002

37. Thike AA, Cheok PY, Jara-Lazaro AR, Tan B, Tan P, Tan PH (2010) Triple-negative breast cancer: clinicopathological characteristics and relationship with basal-like breast cancer. Modern pathology : an official journal of the United States and Canadian Academy of Pathology, Inc 23:123-133. doi: 10.1038/modpathol.2009.145

38. Vaklavas C, Forero-Torres A (2011) How do I treat "triple-negative" disease. Current treatment options in oncology 12:369-388. doi: 10.1007/s11864-011-0168-y

39. Vela I, Morrissey C, Zhang X, Chen S, Corey E, Strutton GM, Nelson CC, Nicol DL, Clements JA, Gardiner EM (2013) PITX2 and non-canonical Wnt pathway interaction in metastatic prostate cancer. Clinical & experimental metastasis. doi: 10.1007/s10585-013-9620-7

40. Wolff AC, Hammond ME, Schwartz JN, Hagerty KL, Allred DC, Cote RJ, Dowsett M, Fitzgibbons PL, Hanna WM, Langer A, McShane LM, Paik S, Pegram MD, Perez EA, Press MF, Rhodes A, Sturgeon C, Taube SE, Tubbs R, Vance GH, van de Vijver M, Wheeler TM, Hayes DF, American Society of Clinical Oncology/College of American P (2007) American Society of Clinical Oncology/College of American Pathologists guideline recommendations for human epidermal growth factor receptor 2 testing in breast cancer. Archives of pathology & laboratory medicine 131:18-43. doi: 10.1043/1543-2165(2007)131[18:ASOCCO]2.0.CO;2

## Claims

1. A method for predicting a triple-negative breast cancer (TNBC) patient’s response to anthracycline-containing poly-chemotherapy, said method comprising:

i) contacting genomic DNA isolated from a biological sample of said TNBC patient with at least one reagent, or series of reagents, that distinguishes between methylated and non-methylated CpG dinucleotides;
ii) determining, based on such contacting of i), a methylation state of at least one CpG dinucleotide sequence of a paired-like homeodomain transcription factor 2 (PITX2) gene and/or of one or several regulatory sequences thereof within said biological sample, and
iii) making a prediction of said TNBC patient's response to anthracycline-containing polychemotherapy based on the determined methylation state.

**2.** The method according to claim 1, wherein said polychemotherapy is adjuvant polychemotherapy.

**3.** The method according to any of claims 1 and 2, wherein said prediction of said TNBC patient's response to anthracycline-containing polychemotherapy is based on whether the determined methylation state of said PITX2 gene and/or of one or several regulatory sequences thereof exceeds a defined threshold, wherein said prediction is made in terms of one or several parameters selected from disease-free survival (DFS), metastasis-free survival (MFS) and overall survival (OS), respectively, and wherein said prediction is negative, if the determined methylation state is equal to or does not exceed said threshold, and wherein said prediction is positive, if said determined methylation state does exceed said threshold.

**4.** The method according to any of the foregoing claims, wherein said contacting in step i) comprises contacting genomic DNA isolated from said biological sample obtained from said patient with at least one reagent, or series of reagents that distinguishes between methylated and non-methylated CpG dinucleotides within at least one target region of the genomic DNA, wherein the target region comprises or hybridizes under stringent conditions to a sequence of at least 16 contiguous nucleotides of the PITX2 gene and/or of regulatory sequences thereof, wherein said contiguous nucleotides comprise at least one CpG dinucleotide sequence.

**5.** The method according to any of the foregoing claims, wherein said one or more reagents comprise bisulfite, hydrogen sulfite, disulfite or combinations thereof.

**6.** The method according to any of the foregoing claims, wherein the step of determining the methylation state is performed by oligonucleotide hybridization analysis, methylation-sensitive single-nucleotide primer extension (Ms-SNuPE), sequencing, quantitative real time PCR or oligonucleotide array analysis.

**7.** The method according to any of claims 1 - 6, wherein said determination of said methylation state in step ii) is or comprises the determination of a methylation score of said PITX2 gene or of one or several regulatory sequences thereof in said biological sample, and wherein prediction of said TNBC patient's response to said anthracycline-containing polychemotherapy is based on whether the methylation score in said biological sample exceeds a defined threshold methylation score, wherein said prediction is made in terms of one or several parameters selected from disease-free survival (DFS), metastasis-free survival (MFS) and overall survival (OS), and wherein said prediction is negative, if the methylation score of said sample is equal to or does not exceed said defined threshold methylation score, and wherein said prediction is positive, if said methylation score of said sample does exceed said defined threshold methylation score,
wherein said methylation score in said biological sample is determined according to the formula:

$$\text{methylation score in biological sample} = \frac{100}{1+2^{(CT\ methylated - CT\ unmethylated)}}$$

wherein "CT methylated" is the cycle threshold value of methylated PITX2 and/or of methylated regulatory sequence(s) thereof,
and wherein "CT unmethylated" is the cycle threshold value of unmethylated PITX2 and/or of unmethylated regulatory sequence(s) thereof.

**8.** The method according to any of claims 1-7, comprising the steps:

i') isolating genomic DNA from a biological sample taken from said patient and treating said genomic DNA, or a fragment thereof, with one or more reagents, in order to convert 5-position unmethylated cytosine bases to uracil or to another base that is detectably dissimilar to cytosine in terms of hybridization properties, thus producing treated genomic DNA; contacting said treated genomic DNA or said treated fragment thereof, with an amplification enzyme and at least two primers comprising, in each case, a contiguous sequence of at least 16 nucleotides in length that is complementary to, or hybridizes under stringent conditions to a sequence of the PITX2 gene and/or to one or several of its regulatory sequences thereof, and/or to complements thereof, wherein said at least two primers hybridize to said sequence(s) only if such sequence(s) has(have) been treated with said one or more reagents, such that the treated DNA or a fragment thereof is amplified to produce one or more amplificates, and wherein during amplification there are additionally at least two PITX2-specific probes present that distinguish between methylated and unmethylated PITX2 sequences, said at least two PITX2-specific probes producing two signals being indicative of methylated and unmethylated PITX2 sequences, respectively, said signals being proportional to the amount of methylated und unmethylated PITX2-sequences present in said biological sample, respectively,

ii') determining, based on said signals of methylated und unmethylated PITX2-sequences a methylation score of said PITX2 gene in said biological sample,

wherein said methylation score in said biological sample is determined according to the formula

$$\text{methylation score in biological sample} = \frac{100}{1+2^{(CT\ methylated - CT\ unmethylated)}}$$

wherein "CT methylated" is the cycle threshold value of methylated PITX2 and/or of methylated regulatory sequence(s) thereof,

and wherein "CT unmethylated" is the cycle threshold value of unmethylated PITX2 and/or of unmethyated regulatory sequence(s) thereof,, and

iii') making a prediction of said TNBC patient's response to anthracycline-containing polychemotherapy based on said determined methylation score in said biological sample, and wherein prediction of said TNBC patient's response to said anthracycline-containing polychemotherapy is based on whether the methylation score in said biological sample exceeds a defined threshold methylation score, wherein said prediction is made in terms of one or several parameters selected from disease-free survival (DFS), metastasis-free survival (MFS) and overall survival (OS), and wherein said prediction is negative, if the methylation score of said sample is equal to or does not exceed said defined threshold methylation score, and wherein said prediction is positive, if said methylation score of said sample does exceed said defined threshold methylation score.

**9.** The method according to any of claims 1-8, further comprising

iv) determining a suitable treatment regimen for said patient, wherein such suitable treatment regimen for said patient is a treatment with polychemotherapy including one or several anthracyclines if said prediction of step iii) or iii') is positive, and said suitable treatment regimen is a therapy excluding anthracycline treatment, if said prediction is negative.

**10.** The method according to any of claims 7-9, wherein said defined threshold methylation score is in the range of from 4 - 10, particularly in the range of from 5 - 8, more particularly 5- 7, even more particularly 6 - 7, most particularly approximately 6.35 +/-0.3.

**11.** The method according to any of the foregoing claims, wherein said biological sample is selected from the group consisting of breast cancer-derived tumor cell lines and breast cancer tissues, e.g. presurgical core biopsies, biopsies taken at time of primary surgery, circulating peripheral breast cancer tumor cells, tumor-afflicted lymph nodes, metastases, in particular in the frozen state or fixed with any fixative and then embedded in paraffin, bodily fluids such as nipple aspirate, blood, serum, plasma, urine or any other bodily fluid, and combinations thereof.

**12.** The method according to any of the foregoing claims, wherein the PITX2 gene has a sequence represented by SEQ ID NO:1 or SEQ ID NO:2.

**13.** The method according to any of claims 8 - 12, wherein said sequence which said at least two primers are complementary to or hybridize thereto has a sequence represented by SEQ ID NO: 3 and/or 4, and/or wherein said sequence of the PITX2 gene before treatment with said one or more reagent(s) has a sequence represented by SEQ ID NO:5, and/or wherein said at least two primers have sequences represented by SEQ ID NO: 6 and 7; and/or wherein said at least two PITX2-specific probes have sequences represented by SEQ ID NO:8 and SEQ ID NO:9.

**14.** The method according to any of claims 8 - 13, wherein said at least two PITX2-specific probes are Taqman hydrolysis probes that distinguish between methylated and unmethylated PITX2 sequences, wherein a first Taqman hydrolysis probe is specific for methylated PITX2 sequence(s) and produces a first signal upon amplification of methylated PITX2 sequence(s), and wherein a second Taqman hydrolysis probe is specific for unmethylated PITX2 sequence(s) and produces a second signal different from said first signal, upon amplification of unmethylated PITX2 sequence(s), said first and said second signal preferably being fluorescence signals.

**15.** Use of a PITX2 gene, a PITX2 regulatory sequence, or a stretch of at least 16 contiguous nucleotides of the foregoing sequences, or of a complementary sequence thereto or of a sequence that hybridizes under stringent conditions to any of the foregoing sequences, in a method for predicting a triple-negative breast cancer (TNBC) patient's response to anthracycline-containing adjuvant polychemotherapy according to any of claims 1-14.

**16.** The use according to claim 15, wherein said PITX2 gene, said PITX2 regulatory sequence, said stretch of at least 16 contiguous nucleotides of the foregoing sequences, said complementary sequence thereto, or said sequence that hybridizes under stringent conditions to any of the foregoing sequences, is selected from any of the sequences represented by SEQ ID NO:1 - 9.

*Fig. 1a: ABCS – Disease-free Survival by PITX2 DNA Methylation*

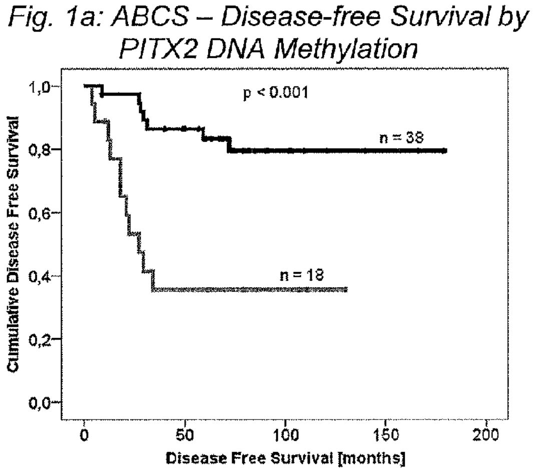


*No. of patients at risk after (x) months:*
***High methylation**: 36 (24), 26 (60), 4 (120)*
***Low methylation**: 9 (24), 6 (60), 1 (120)*

*Fig. 1b: ABCS – Metastasis-free Survival by PITX2 DNA Methylation*

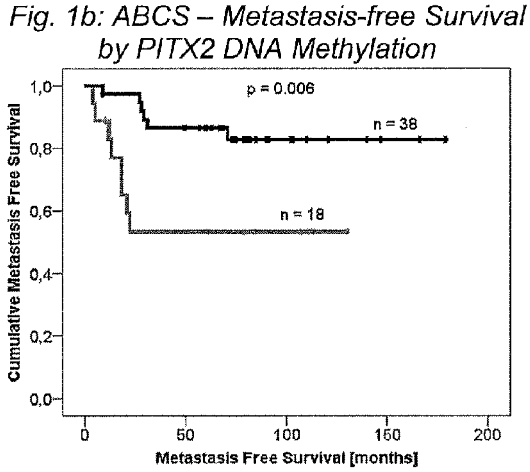


*No. of patients at risk after (x) months:*
***High methylation**: 36 (24), 28 (60), 5 (120)*
***Low methylation**: 9 (24), 9 (60), 1 (120)*

*Fig. 1c: ABCS – Overall Survival by PITX2 DNA Methylation*

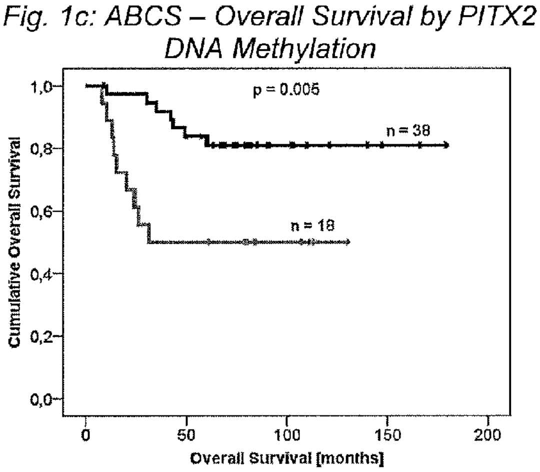


*No. of patients at risk after (x) months:*
***High methylation**: 36 (24), 28 (60), 5 (120)*
***Low methylation**: 11 (24), 9 (60), 1 (120)*

Figure 1

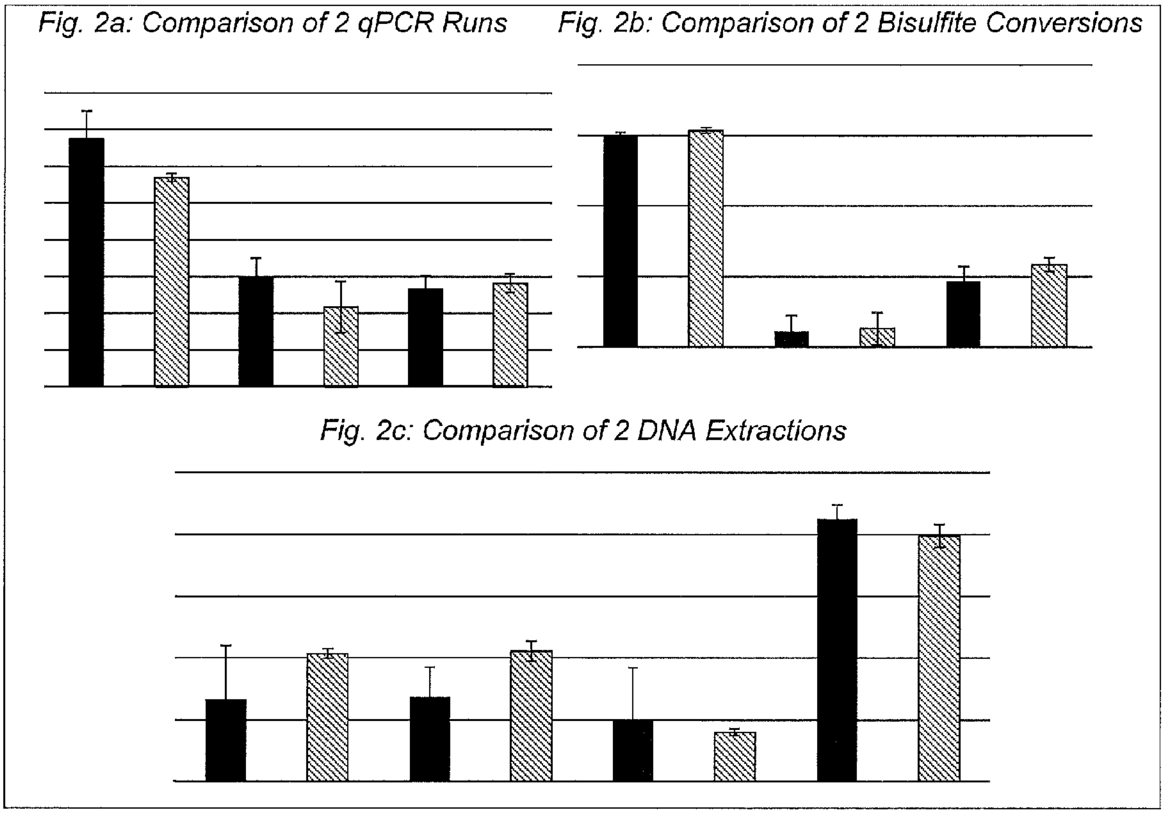
Fig. 2a: Comparison of 2 qPCR Runs
Fig. 2b: Comparison of 2 Bisulfite Conversions
Fig. 2c: Comparison of 2 DNA Extractions

Figure 2

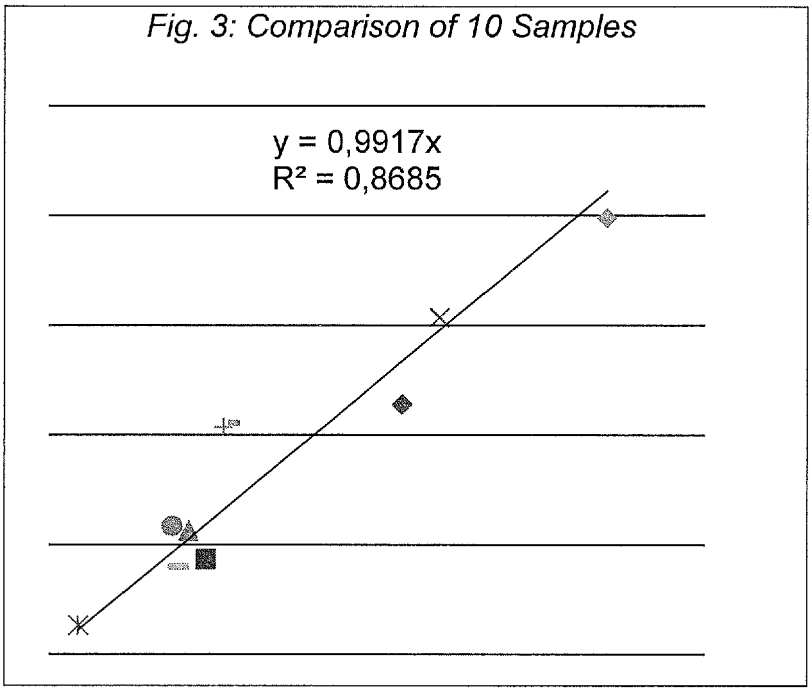


Figure 3

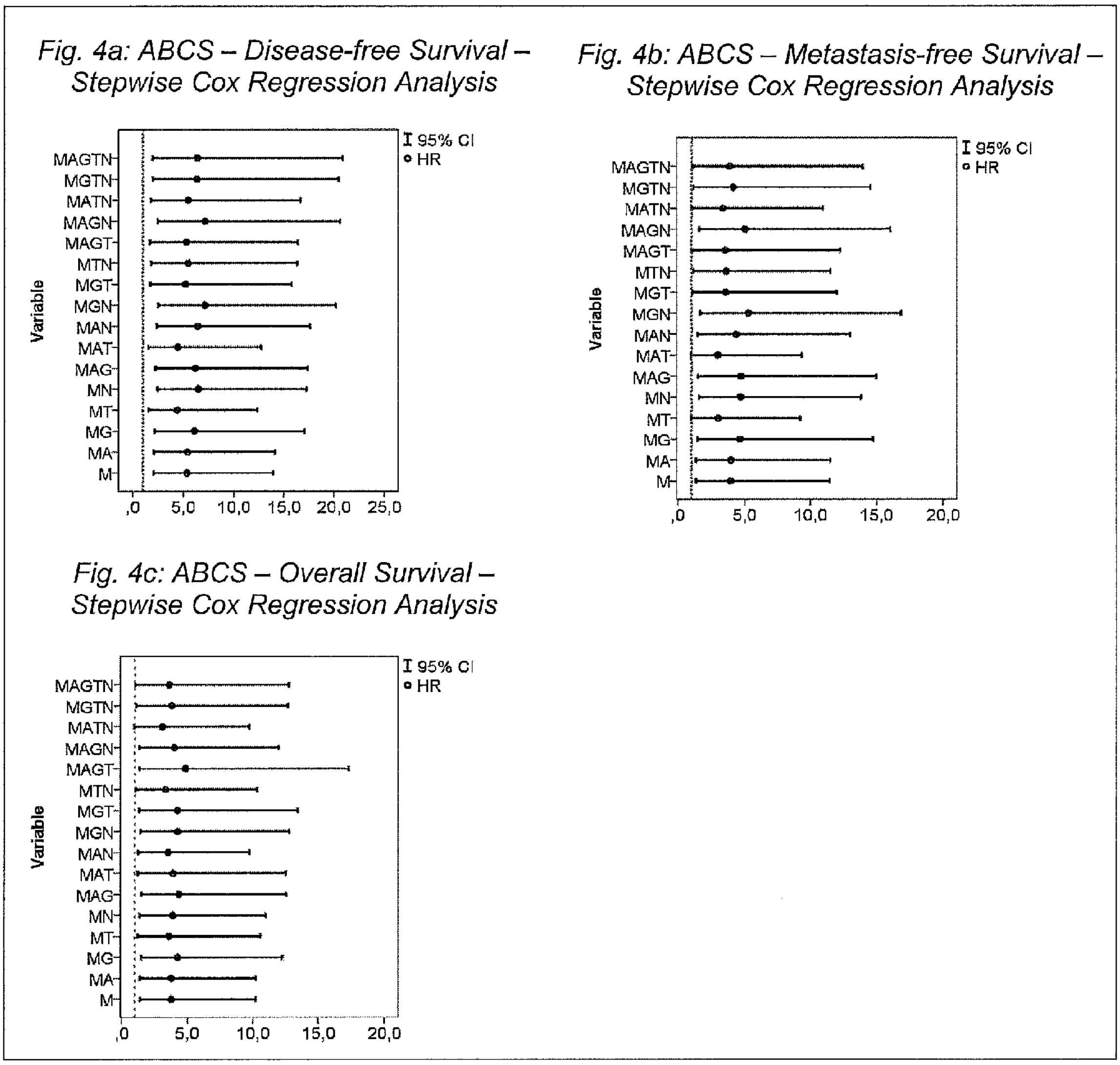
Fig. 4a: ABCS – Disease-free Survival – Stepwise Cox Regression Analysis
Variable
MAGTN
MGTN
MATN
MAGN
MAGT
MTN
MGT
MGN
MAN
MAT
MAG
MN
MT
MG
MA
M
,0
5,0
10,0
15,0
20,0
25,0
I 95% CI
o HR
Fig. 4b: ABCS – Metastasis-free Survival – Stepwise Cox Regression Analysis
Variable
MAGTN
MGTN
MATN
MAGN
MAGT
MTN
MGT
MGN
MAN
MAT
MAG
MN
MT
MG
MA
M
,0
5,0
10,0
15,0
20,0
I 95% CI
o HR
Fig. 4c: ABCS – Overall Survival – Stepwise Cox Regression Analysis
Variable
MAGTN
MGTN
MATN
MAGN
MAGT
MTN
MGT
MGN
MAN
MAT
MAG
MN
MT
MG
MA
M
,0
5,0
10,0
15,0
20,0
I 95% CI
o HR

Figure 4

Europäisches Patentamt
European Patent Office
Office européen des brevets

# EUROPEAN SEARCH REPORT

Application Number
EP 16 17 0625

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PILLAI SREERAJ G ET AL: "Paired-like Homeodomain Transcription factor 2expression by breast cancer bone marrow disseminated tumor cells is associated with early recurrent disease development", BREAST CANCER RESEARCH AND TREATMENT, SPRINGER , NY, US, vol. 153, no. 3, 23 September 2015 (2015-09-23), pages 507-517, XP035551288, ISSN: 0167-6806, DOI: 10.1007/S10549-015-3576-Z [retrieved on 2015-09-23] * page 509, right-hand column; table 1 * * page 513 - page 516 * | 1-16 | INV. C12Q1/68 |
| A | WO 2015/169857 A1 (UNIVERSITÉ LIBRE DE BRUXELLES [BE]) 12 November 2015 (2015-11-12) * claim 1; figure 8 * | 1 | |

TECHNICAL FIELDS SEARCHED (IPC)

C12Q

The present search report has been drawn up for all claims

1

| Place of search | Date of completion of the search | Examiner |
|---|---|---|
| The Hague | 9 November 2016 | Gabriels, Jan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO.

EP 16 17 0625

This annex lists the patent family members relating to the patent documents cited in the above-mentioned European search report. The members are as contained in the European Patent Office EDP file on
The European Patent Office is in no way liable for these particulars which are merely given for the purpose of information.

09-11-2016

| Patent document cited in search report | Publication date | Patent family member(s) | Publication date |
|---|---|---|---|
| WO 2015169857 A1 | 12-11-2015 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **SAMBROOK et al.** Molecular Cloning, A Laboratory Manual. Cold Spring Harbor Press, 1989
- Current Protocols in Molecular Biology. John Wiley & Sons, 1995
- **KREBS.** Deutschland. 2007
- Gesellschaft der epidemiologischen Krebsregister in Deutschland. 2012
- **ALBERGARIA A ; RICARDO S ; MILANEZI F ; CARNEIRO V ; AMENDOEIRA I ; VIEIRA D ; CAMESELLE-TEIJEIRO J ; SCHMITT F.** Nottingham Prognostic Index in triple-negative breast cancer: a reliable prognostic tool?. *BMC cancer,* 2011, vol. 11, 299
- **BANEZ LL ; SUN L ; VAN LEENDERS GJ ; WHEELER TM ; BANGMA CH ; FREEDLAND SJ ; ITTMANN MM ; LARK AL ; MADDEN JF ; HARTMAN A.** Multicenter clinical validation of PITX2 methylation as a prostate specific antigen recurrence predictor in patients with post-radical prostatectomy prostate cancer. *The Journal of urology,* 2010, vol. 184, 149-156
- **BASU M ; ROY SS.** Wnt/beta-catenin pathway is regulated by PITX2 homeodomain protein and thus contributes to the proliferation of human ovarian adenocarcinoma cell, SKOV-3. *The Journal of biological chemistry,* 2013, vol. 288, 4355-4367
- **BAUER KR ; BROWN M ; CRESS RD ; PARISE CA ; CAGGIANO V.** Descriptive analysis of estrogen receptor (ER)-negative, progesterone receptor (PR)-negative, and HER2-negative invasive breast cancer, the so-called triple-negative phenotype: a population-based study from the California cancer Registry. *Cancer,* 2007, vol. 109, 1721-1728
- **DENT R ; TRUDEAU M ; PRITCHARD KI ; HANNA WM ; KAHN HK ; SAWKA CA ; LICKLEY LA ; RAWLINSON E ; SUN P ; NAROD SA.** Triple-negative breast cancer: clinical features and patterns of recurrence. *Clin Cancer Res,* 2007, vol. 13, 4429-4434
- **ELIAS AD.** Triple-negative breast cancer: a short review. *American journal of clinical oncology,* 2010, vol. 33, 637-645
- **FUNG FK ; CHAN DW ; LIU VW ; LEUNG TH ; CHEUNG AN ; NGAN HY.** Increased expression of PITX2 transcription factor contributes to ovarian cancer progression. *PloS one,* 2012, vol. 7, 37076
- beta-Catenin pathway activation in breast cancer is associated with triple-negative phenotype but not with CTNNB1 mutation. **GEYER FC ; LACROIX-TRIKI M ; SAVAGE K ; ARNEDOS M ; LAMBROS MB ; MACKAY A ; NATRAJAN R ; REIS-FILHO JS.** Modern pathology : an official journal. United States and Canadian Academy of Pathology, Inc, 2011, vol. 24, 209-231
- American Society of Clinical Oncology/College of American Pathologists guideline recommendations for immunohistochemical testing of estrogen and progesterone receptors in breast cancer. **HAMMOND ME ; HAYES DF ; DOWSETT M ; ALLRED DC ; HAGERTY KL ; BADVE S ; FITZGIBBONS PL ; FRANCIS G ; GOLDSTEIN NS ; HAYES M.** Archives of pathology & laboratory medicine. American Society of Clinical O, College of American P, 2010, vol. 134, 48-72
- Multicenter study using paraffin-embedded tumor tissue testing PITX2 DNA methylation as a marker for outcome prediction in tamoxifen-treated, node-negative breast cancer patients. **HARBECK N ; NIMMRICH I ; HARTMANN A ; ROSS JS ; CUFER T ; GRUTZMANN R ; KRISTIANSEN G ; PARADISO A ; HARTMANN O ; MARGOSSIAN A.** Journal of clinical oncology : official journal. American Society of Clinical Oncology, 2008, vol. 26, 5036-5042
- DNA methylation markers predict outcome in node-positive, estrogen receptor-positive breast cancer with adjuvant anthracycline-based chemotherapy. **HARTMANN O ; SPYRATOS F ; HARBECK N ; DIETRICH D ; FASSBENDER A ; SCHMITT M ; EPPENBERGER-CASTORI S ; VUAROQUEAUX V ; LEREBOURS F ; WELZEL K.** Clinical cancer research : an official journal. American Association for Cancer Research, 2009, vol. 15, 315-323
- **HIROSE H ; ISHII H ; MIMORI K ; TANAKA F ; TAKEMASA I ; MIZUSHIMA T ; IKEDA M ; YAMAMOTO H ; SEKIMOTO M ; DOKI Y.** The significance of PITX2 overexpression in human colorectal cancer. *Annals of surgical oncology,* 2011, vol. 18, 3005-3012
- **HOTHORN T.** maxstat: Maximally Selected Rank Statistics. *In: R package,* 2011
- **JOVANOVIC J ; RONNEBERG JA ; TOST J ; KRISTENSEN V.** The epigenetics of breast cancer. *Molecular oncology,* 2010, vol. 4, 242-254

- **KHASRAW M ; BELL R ; DANG C.** Epirubicin: is it like doxorubicin in breast cancer? A clinical review. *Breast,* 2012, vol. 21, 142-149
- **KIOUSSI C ; BRIATA P ; BAEK SH ; ROSE DW ; HAMBLET NS ; HERMAN T ; OHGI KA ; LIN C ; GLEIBERMAN A ; WANG J.** Identification of a Wnt/Dvl/beta-Catenin --> Pitx2 pathway mediating cell-type-specific proliferation during development. *Cell,* 2002, vol. 111, 673-685
- **KREIKE B ; VAN KOUWENHOVE M ; HORLINGS H ; WEIGELT B ; PETERSE H ; BARTELINK H ; VAN DE VIJVER MJ.** Gene expression profiling and histopathological characterization of triple-negative/basal-like breast carcinomas. *Breast cancer research : BCR,* 2007, vol. 9, 65
- **LEE WK ; CHAKRABORTY PK ; THEVENOD F.** Pituitary homeobox 2 (PITX2) protects renal cancer cell lines against doxorubicin toxicity by transcriptional activation of the multidrug transporter ABCB1. International journal of cancer. *Journal international du cancer,* 2013, vol. 133, 556-567
- **LUU HH ; ZHANG R ; HAYDON RC ; RAYBURN E ; KANG Q ; SI W ; PARK JK ; WANG H ; PENG Y ; JIANG W.** Wnt/beta-catenin signaling pathway as a novel cancer drug target. *Current cancer drug targets,* 2004, vol. 4, 653-671
- DNA-methylation of the homeodomain transcription factor PITX2 reliably predicts risk of distant disease recurrence in tamoxifen-treated, node-negative breast cancer patients-Technical and clinical validation in a multi-centre setting in collaboration. **MAIER S ; NIMMRICH I ; KOENIG T ; EPPENBERGER-CASTORI S ; BOHLMANN I ; PARADISO A ; SPYRATOS F ; THOMSSEN C ; MUELLER V ; NAHRIG J.** European journal of cancer. European Organisation for Research and Treatment of Cancer (EORTC) PathoBiology group, 2007, vol. 43, 1679-1686
- **MCSHANE LM ; ALTMAN DG ; SAUERBREI W ; TAUBE SE ; GION M ; CLARK GM.** Statistics Subcommittee of the NCIEWGoCD (2005) REporting recommendations for tumor MARKer prognostic studies (REMARK). *Nature clinical practice. Oncology,* vol. 2, 416-422
- **MERSIN H ; YILDIRIM E ; BERBEROGLU U ; GULBEN K.** The prognostic importance of triple negative breast carcinoma. *Breast,* 2008, vol. 17, 341-346
- **MORENO CS ; EVANS CO ; ZHAN X ; OKOR M ; DESIDERIO DM ; OYESIKU NM.** Novel molecular signaling and classification of human clinically nonfunctional pituitary adenomas identified by gene expression profiling and proteomic analyses. *Cancer research,* 2005, vol. 65, 10214-10222
- **NIMMRICH I ; SIEUWERTS AM ; MEIJER-VAN GELDER ME ; SCHWOPE I ; BOLT-DE VRIES J ; HARBECK N ; KOENIG T ; HARTMANN O ; KLUTH A ; DIETRICH D.** DNA hypermethylation of PITX2 is a marker of poor prognosis in untreated lymph node-negative hormone receptor-positive breast cancer patients. *Breast cancer research and treatment,* 2008, vol. 111, 429-437
- **NOVAK P ; JENSEN T ; OSHIRO MM ; WATTS GS ; KIM CJ ; FUTSCHER BW.** Agglomerative epigenetic aberrations are a common event in human breast cancer. *Cancer research,* 2008, vol. 68, 8616-8625
- **OAKMAN C ; MORETTI E ; GALARDI F ; BIAGIONI C ; SANTARPIA L ; BIGANZOLI L ; DI LEO A.** Adjuvant systemic treatment for individual patients with triple-negative breast cancer. *Breast,* 2011, vol. 20 (3), 135-141
- **PEROU CM ; SORLIE T ; EISEN MB ; VAN DE RIJN M ; JEFFREY SS ; REES CA ; POLLACK JR ; ROSS DT ; JOHNSEN H ; AKSLEN LA.** Molecular portraits of human breast tumours. *Nature,* 2000, vol. 406, 747-752
- **PRAT A ; PARKER JS ; KARGINOVA O ; FAN C ; LIVASY C ; HERSCHKOWITZ JI ; HE X ; PEROU CM.** Phenotypic and molecular characterization of the claudin-low intrinsic subtype of breast cancer. *Breast cancer research : BCR,* 2010, vol. 12, 68
- **SCHATZ P ; DIETRICH D ; KOENIG T ; BURGER M ; LUKAS A ; FUHRMANN I ; KRISTIANSEN G ; STOEHR R ; SCHUSTER M ; LESCHE R.** Development of a diagnostic microarray assay to assess the risk of recurrence of prostate cancer based on PITX2 DNA methylation. *The Journal of molecular diagnostics : JMD,* 2010, vol. 12, 345-353
- European Organisation for Research and Treatment of Cancer (EORTC) Pathobiology Group standard operating procedure for the preparation of human tumour tissue extracts suited for the quantitative analysis of tissue-associated biomarkers. **SCHMITT M ; MENGELE K ; SCHUEREN E ; SWEEP FC ; FOEKENS JA ; BRUNNER N ; LAABS J ; MALIK A ; HARBECK N.** European journal of cancer. European Organisation for R, Treatment of Cancer Pathobiology G, 2007, vol. 43, 835-844
- **SHEN C ; HUANG Y ; LIU Y ; WANG G ; ZHAO Y ; WANG Z ; TENG M ; WANG Y ; FLOCKHART DA ; SKAAR TC.** A modulated empirical Bayes model for identifying topological and temporal estrogen receptor alpha regulatory networks in breast cancer. *BMC systems biology,* 2011, vol. 5, 67

- **SORLIE T ; PEROU CM ; TIBSHIRANI R ; AAS T ; GEISLER S ; JOHNSEN H ; HASTIE T ; EISEN MB ; VAN DE RIJN M ; JEFFREY SS.** Gene expression patterns of breast carcinomas distinguish tumor subclasses with clinical implications. *Proceedings of the National Academy of Sciences of the United States of America,* 2001, vol. 98, 10869-10874
- **TAN DS ; MARCHIO C ; JONES RL ; SAVAGE K ; SMITH IE ; DOWSETT M ; REIS-FILHO JS.** Triple-negative breast cancer: molecular profiling and prognostic impact in adjuvant anthracycline-treated patients. *Breast cancer research and treatment,* 2008, vol. 111, 27-44
- **TEAM RDC.** R: A language and environment for statistical computing. *In:R Foundation for Statistical Computing, Vienna, Austria,* 2012
- **THERIAULT RL ; LITTON JK ; MITTENDORF EA ; CHEN H ; MERIC-BERNSTAM F ; CHAVEZ-MACGREGOR M ; MORROW PK ; WOODWARD WA ; SAHIN A ; HORTOBAGYI GN.** Age and survival estimates in patients who have node-negative T1 ab breast cancer by breast cancer subtype. *Clinical breast cancer,* 2011, vol. 11, 325-331
- Triple-negative breast cancer: clinicopathological characteristics and relationship with basal-like breast cancer. **THIKE AA ; CHEOK PY ; JARA-LAZARO AR ; TAN B ; TAN P ; TAN PH.** Modern pathology : an official journal. United States and Canadian Academy of Pathology, Inc, 2010, vol. 23, 123-133
- **VAKLAVAS C ; FORERO-TORRES A.** How do I treat "triple-negative" disease. *Current treatment options in oncology,* 2011, vol. 12, 369-388
- **VELA I ; MORRISSEY C ; ZHANG X ; CHEN S ; COREY E ; STRUTTON GM ; NELSON CC ; NICOL DL ; CLEMENTS JA ; GARDINER EM.** PITX2 and non-canonical Wnt pathway interaction in metastatic prostate cancer. *Clinical & experimental metastasis,* 2013
- American Society of Clinical Oncology/College of American Pathologists guideline recommendations for human epidermal growth factor receptor 2 testing in breast cancer. **WOLFF AC ; HAMMOND ME ; SCHWARTZ JN ; HAGERTY KL ; ALLRED DC ; COTE RJ ; DOWSETT M ; FITZGIBBONS PL ; HANNA WM ; LANGER A.** Archives of pathology & laboratory medicine. American Society of Clinical Oncology/College of American P, 2007, vol. 131, 18-43

SEQUENCE LISTING

<110> Klinikum rechts der Isar der Technischen Universität München
Helmholtz Zentrum München

<120> A method for predicting a breast cancer patient's response to anthracycline treatment

<130> U30674EP

<160> 9

<170> PatentIn version 3.5

<210> 1
<211> 26930
<212> DNA
<213> Homo sapiens

<400> 1
gggctcttga tgcaatctat atgccaatct acttcattag ttcttctttt atttacagtt 60

tggtaaagaa tatgggtgga gctgttctgg gctcacttgc acacatgtcc aaactgcttt 120

gaaaaaggaa gggcaagaaa gagtggtatc caagttggaa tcaggcaggc atttcagatc 180

aagagacgaa ctggaaaggg aacatctgtt agataccctg ggtttgaagg cagtctgtgt 240

aagttttcat atctctgagt gtgtgcacac agtggagagg gtggagcctg ccatcctcaa 300

atctgaaaag attgagagat ttcagagggc ccagatgtgc caaaggtcag agggatcaat 360

atacaggccc taccacggaa aggcggggaa aaggttcgaa tagaaaactg ctgcagaagg 420

gaagccactg agaggtaagg gagtttctga ataattaaaa agttaagaat aagcaaaagg 480

aaggaggtcg ggtgggggat aaaaaaaagc agttgatgtg gtaattaaga atttggtggg 540

agcctgggca ggtcacctcc tttctcagat cagagcccca tcagaaattc tttcaagtgt 600

ccttctgcgt cgccaaagat gacaacagca aatcaataag tgcttgaaat gaaaggggat 660

gttgactagc cccaggcta cagatttccc gccgccagcc ttttctgaac tcctatagcg 720

tgcctttgca ccgcctctct taagaagagc taccttttat tcctattctc aggacgaagg 780

taagtgctca gttagcatat ctattaaatg tcagctttgg ttccagctct ccgtttgcgc 840

ggaaagctca ctgccatagc gcgcccagcc tgccggaggg gcagacagaa aaagcaagct 900

tggcctggcg acttgcgggg ccacgcacct ccagggctgg cccggagtct tccagagttt 960

aacgctcctg ggttagaact gtaaggtccc ggtccgagca aagggcctga gccaccgtag 1020

ccgtgggagc gttccttcca cttgaatgca ctcactcaca aacaagcaca aaatcttttt 1080

aacagcagag gagaaagacc cctgctccaa aattaaagct gggaatcacc ggaaactccg 1140

ttttgagtgc gagatattgg tctgttacct ttctaatctt catatccctc ctgtaatatg 1200

tcttgattta acaatctttc cagcgcccag cattgcccgg acgttttaat tgggtaattc 1260

attagtgagt caatacaggc atttatatat tcttttagct caagtggtta agtactaatt 1320
tcgaaatgat tataaaacac cggaatcggc aacgcatagt aattttaatt tatatgcaaa 1380
tcaattggtt catcttaaat gccttttttta aaaaaacaat tattgtattg tagcatcgga 1440
ggcatggatc aaacctctag aatagacaat tcggaaacaa gacctggact aggaagacaa 1500
tttagaacag ccaacaaatc aataatgttt gaggcagtta aacatcgcta gccattggta 1560
tttacatact cgcttgttgt cagataagga gctggggaaa ttgcttgcca gggttgagat 1620
cataatccag agtgaagaaa gtaaatggta gcacacagcc cgtcacagcg ggctctgaaa 1680
taatactgta cctttcccaa atcctgactc ttgggtgaca gggagttggc ggaggtcacc 1740
ccacatttgt ccacggtttt gccccaattt gatcacgaaa ttgttgttct ccctgagctt 1800
tccaatttga ttaccattct aacggttctg tcacttgtct caacatattg gggggaggag 1860
tgtaattgag attctcatta aaaattatct gaacccactt agccagcact gtttcatcta 1920
agcttagttt tatgggctgt atttaattcc ctgtgccccc cacacattaa aatcagatca 1980
tcaaaatgtc ggtaggaaag ggtgaaggaa atggtccaat gctccagttt actggaagac 2040
tattatcttt agacatagtt caaaattttg aggaaataaa aaggatatac gctttggggg 2100
gaaaatgttt taatattcta gaatgggggt attactcccc ccattccaga gaatccgcac 2160
tggagttgtt tatgtaaaaa tgtaacatcc ttgaaattca cagatacgta aggttagtgt 2220
ctccccttcc ccaggctccc agtccaggcg atctagccct aaaggagcta gtacctttga 2280
tgctacaatc ttgtttacat ctgcagggca gagaattgct tgctttgctt ggacgctccc 2340
tccacccccct tctaatttga agtaatcgga atctaaatac agtcgccaag gcccgctctt 2400
cctttactgc tttgacaagg gaaaaacctg aaatccacgt cttaaatcag ctcggtggtt 2460
tgtagccccc cagcaccctg cttctacgat tgcatgccta atgtattccc tggtgattct 2520
gggcattaat tagttgttta ataggagtat gactaaaaat gtaaaagaag gattaggagc 2580
gtgaaacgta tgtccagctc cttccacaca ctcgaggagg gaatgagaat cattctgtat 2640
cttctatttc tccaggagcc atttgcattt cccaccagct gctcacttca gctgcactgg 2700
cgctgggcaa ggcgaggacc caaaagctca gcgcagtgtc tgcggcggcc gggactgggg 2760
ttaaccagcc tctggcgggc gagactccag acagaagggg ggcgagagga acgtgagctt 2820
cccgagcccc ttcctctcag ccctggtttg caaacctctg aaacctgaaa ggggagggag 2880
ttgcacgcgc gtatctttgc gtcttttcag cgcaactccc ttccctctcc ctgtgtctct 2940
ccgcggatct ctgaatcttt ctgtctttgg ttttctcatt ctcttccaac ttttccatga 3000
gattgcctat cctcgccacc agctgaaggc aaggccgttc tgctacgagc gcctcttaat 3060
ctctacaaaa tgaaaagaaa aaaagggagg attattagcc cattactcag aggaatgggg 3120
aggctgcaaa aatcgtcgat gggcagaggt gaagatgtct ttctcggact gcactttccg 3180

gtgtcctgta actagagttc agttgtggga cttgttgaag aaatttgatt ttcttgcctc 3240
ggcgagattt caaaaaccag aaatagaaat tctcagagtc agagaggaaa tacaattaaa 3300
cagcacgtgg gcattttccc cctcatttct ctccccttaa ataacactgc tttgagtttc 3360
cactgggtaa agagagaaag tttgagtttt cacggatgtt acgtggaggt tagaaatggc 3420
ttaaaatgta gatctctaat cagttttctt cgtggctgaa gaggctaacc ctttccataa 3480
aatgagtcca tctgtcgact gttagctatt tcaaagtgaa gggatttagc actcaaaaca 3540
aattgagcaa gtttgtttgc ctgtttttac tgctaactca aatgaattca aaacacggag 3600
taattcaaga aaacacataa catgttccag acagccccca aaagtaggga aagcccagca 3660
cctatatagt gactagggtt agttttaagc gccaagcttt tttaaacgta tctattttat 3720
gcacattctc ccgagtcact atatatttct aaaattgcga gtattggtat attgatttag 3780
gaagagcaat acaactttta gagggaactt tattctcaat tagggaccaa agagatgtct 3840
ttttaatagc gggcctgagt tttgctctca agcaggaatt aatattggtg ggaaaatccg 3900
aatccaggag caatggctgt gttccggcac tttccaaaaa catacattaa caggatgccc 3960
ttgagattga aaaaacattg tcccatatgc ctggcagaag ccttcacacc tggtcctcca 4020
ggcgaattat atttatagtc cttccactca gaggcaggac agagccaaaa tattctgctc 4080
actaccaaaa tacacatctt tgctcaagtc aagaaatcag aaaatcaggg ttcagaagta 4140
aggcacactt ttcgagtgag aatatgccct gtaatttcac atactctttg ctttgcagga 4200
gcaaatgtgg acttgaggga aactctctcc cccaccccca cttctatccc gtgcaattta 4260
ataccatcct cgccaggaac cttaacctcg tcattttaaa aaatgagata tccgtgaccc 4320
agggtgaact tgttgaatgt aggtacagca gaggaaattc tagactctat gagcgtctga 4380
gccttgtcca gtgcaaaccc ttcgtgaaca ctgggtcagt gcgtggccgt gcccacctgt 4440
gcgccgacac tctcagcatg cctggtccac ccgccttgac ctcgggcgcg gtgtcccagc 4500
taagctgggc ccagcgtccc ggccttcccc agctgacaag cctagctcgt tcgctcccgg 4560
ctgtggccct cccaccctct cccactagct cactccattc ttctagattt ctcttcactc 4620
atcctctccc atccccaccg cgcccacctc cactcccgcc ctctaccggt ctctcacttt 4680
cctccctccg cagtccctct ttgctgtgac ctctttcctc aactctgcag gcctgaaaga 4740
aggtcacaca cgcacgctca cacccacact ccacacgcct cgtcccaaac aaccccatga 4800
acattgtcct ttgttccgtc tcttgggcca ctttccctgt cgcttcctcc cagcccgtcc 4860
tgatttgctc cccaaaagta cgtttctgtc tccccgctgc cctggcgctc cccctttgat 4920
ttattagggc tgccgggttg gcgcagattg ctttttcttc tcttccatcc catcctccct 4980
tctggtcctc ctttccacag tgggagtccg tgctcctgct cctcggttgg ctcctaagtg 5040

```
ccccgccagg tcccctctcc tttcgctctc ccggctccgg ctcccgactc ttcggcccgc     5100
tggcatctgc ttccctcccc tgcctcgttt ctcgtcgccc ctgctcgctc cccccggcgc     5160
tcgcccgggc gctgtgctcg ctcctggatc gccagccgcg cagccgggct cggccggccg     5220
cccgcgcgcc actgtgcagt ggagtttggt ggaatctctg ctgacgtcac gtcactcccc     5280
acacggagta ggagcagagg gaagagagag ggatgagagg gagggagagg agagagagtg     5340
cgagaccgag cgagaaagct ggagaggagc agaaagaaac tgccagtggc ggctagattt     5400
cggaggcccc agtgcacccg tggactcctt cggaacttgg caccctcagg agccctgcag     5460
tcctctcagg cccggctttc gggcgcttgc cgtgcagccg gaggctcggc tcgctggaaa     5520
tcgccccggg aagcagtggg acgcggagac agcagctctc tcccggtagc cggtaagtgg     5580
aggccatcta tcccgcaggg atgtgagata atgcgagtct ggaaatttgt tccacttcgg     5640
agaatcttca ccgtaggtga tttgtggctt ttggggctaa gtttcgccca aggtaacgca     5700
gtcggcaaac agaccttgca aagccctgtt cctttcgtcc cccgccacag acactaacaa     5760
tctacagggt gctgaagtcg agagggaagc cagaccgtgg ctggcattta aaacgaggta     5820
tcttccctta aatctcggtg ccaacactgc aggaacaaat cctcgggcca aggattagca     5880
ttctcaagat aaagggctgg gtacaaagtt tcagctactg gaagattagc cccccttccca     5940
ttgttatcca ttgggaaaaa aaagaaaaga aaaagattcc atcttaactg gcagttagtg     6000
acctctcagg cccaagcgaa ttacctggga gccaggcctg gatgccaagc tctcaccatt     6060
tctttggatt gtaactcctt taaattgatc accagtcaac tccaatctgg cacttcagga     6120
gatacacttt aaatggatgc agagaattat tttccagctg gagattaaga aaaaaatttt     6180
cgattctaaa cctccgaaat atgttcctct tttccagttt aaccacttta ctttcttaag     6240
caatttagaa atcaaactat cataaggtgg tgtgattttt ttttactctt ttgtgtgagt     6300
attgtcttac taaactaaac ggaaaaaact tttaccatta taaatgtaaa tatcagaatt     6360
catacattct aaaatatttt tatgaaaaat taatctgatt taaagaaatt tccttgcatt     6420
tgttttagtc tatcaatcaa aactaaagat gcttttatca cacaaaatat cattttggca     6480
gaaatccatc taaaattcaa ataccaataa tatcaagaaa acaaagcaca taagcaaaat     6540
aaattgaaga tttttgttga tgtaacatga gcatacaaca tttcaataac caaactttcc     6600
ctaaaaaatt aaatagccac ttcatttgtg gaatgtttta ctttaactca gcaaaattac     6660
acttaaatta tttaggtgct ttgttcctta agttaagcgt gtttgtcttc aaatgttcct     6720
aaagcactta tattaattgg ttgtaaagaa cgcatacaca tggtaaaata cagaactgaa     6780
ctgagcagta ttttaatttc cttaaataat tacttactac aaattaattt actggctaat     6840
ttcacaattt agttcattta aaacacatgt tcctgtgctg tttattttta aactttccat     6900
taaagatttt gttatggggt aacaaagtgt atgaaaaggg gggaaatgtg aaaggatctg     6960
```

ggattattcg aactgtattt ttcctgcact ttcagtcttg cggtagtcat cagaaattat 7020

tttttagcaa attgttttat ttcttagggc ttgcctgcct gctttgccat ggttcctcgt 7080

cctccgttag ccgtgtagtg ctttttgtgt gctcacaata taaaacccaa gttggccaaa 7140

acaagagtcc ttggcatata cattccaact agaacatgaa ctttgggggt gagaactacc 7200

tcccatcagg aaaagtctcc catctcaatt tgtgagatta gccattgaag ccagttccga 7260

agtctggcag ccaaatttct cacagaagac ttgtcttgat agggcaagtt taaggatcag 7320

caggcgggaa ttggaggtct ctttttaaaa aattatcttc cccagttatt tagactcagt 7380

tcttctagta ggcctggtca ttaaatgaag cataaaaatg caagtctcaa ggctcatttt 7440

gactgcaaaa taaatctcca agtcacaagg acatgtagga gtgagctaag gaacacgcct 7500

tgaccttctt ttcagtcctt agagtggagc tctatgagtt cttgaagatt tgttttgtat 7560

tgctttgttt ggtcttcagc actgaagcac ggggaagtgg ggggaagaat gtgtaataat 7620

tgactgactt tacaccaagc aacgcaatct ttttcttttg tatatttcat tctttaaaaa 7680

aaataaataa ataaaaacta tttgcagtta ccatctgcag tgctccggct accagctaat 7740

aatgcagcca gttcagacat ataaaaaaaa aagattatcg aaatgatgat gacatgcaaa 7800

tttcctccga aattatcata agtaaacatt tgaagtctgg actaataaaa tcccatctgt 7860

gttacttcat atcgagttag tagaaagctg tgataatgaa ttttgtaata tctcacgaac 7920

agacatctca atcagggact aatcctgtga ttttactgca gaatcactaa atctggagcc 7980

gccaaactgc tacttctggg cccacgggcc cacaaggatc gaatcggcag agtccccgcc 8040

cgcgttctcg ctagcgggtg ggggaaccgc ctggccgtcc ccaccctgga tccccacgcc 8100

acagcgccgg gcagcccctc ctgtaggcag cgaccttggc cagaggctcc ccagggccca 8160

gctcccttca ggagaggccg agacgcaggg aaacggtact caggccagag gcaggcccgc 8220

agctccctgc cccgcctctg tgcctccgcc aacccgacaa cgcttgctcc caccccgatc 8280

cccgcacccg cgcgaagtgg gccctccggt cgtcggcgta ccctggttag cgtggagaga 8340

ggcaggcgct gagatcgaag gggcctaggg agccctggac cttcttttct tgtctttaaa 8400

gcaaccgcgg ctcttctacc cacccggtgg agcccctcga gacccacctc tcccggcttg 8460

cctgtggcag agaaggggga gcgcgttaaa tgcttggctc gctgcgttgt ggttgaaaac 8520

gtgaaaaaga tttggctcgc ccgggagaga aagggggaga actgggtagc agttatacca 8580

gagctatttc tccgtccttg gcgggcagta aaccctccaa gaacgtttgc cctgctctcc 8640

tcagtctcgc tcagtccacc cagtgtctct cctctgcgat cttaaatcat actttagggt 8700

aattatttgt agtaagtaaa taaatggccg ggttagtatc tctaggagaa agtgtggcta 8760

aatatggaaa agtggctcct gatggatgag aggcccgaac tcagctcgct cctgaaacac 8820

cctaggccaa gagcccgttc gtttcagaat tacagaaaac cgagggaaac tgctgtctag 8880
gacaggggca cgttggcgct gatgttctac aaatgtttac cgagctccaa ctaatggaca 8940
agcactgaag ggtggtcttt gcatacagct tcccaaagag aaaagtcctc tccacccacc 9000
cactcccgct gccattgcgt tcagatgagt tcttaacccc ggcaccgaga ttcttgaaag 9060
taggtccaca gcctccccag cacactgtgg ctttatagtc ctctaacctc tgggcacttt 9120
tgcggcaact ctggagggag atcccctctt gataaataaa tgtcctgggc ccgaggctag 9180
gctggagatg ctgctgcaca gccagaggct gtcaggtcgg aaaaacacgc ctgaagccta 9240
gcacacagta ggcgcctaac agctagtgta acgtagtctc atctgagccc tgctcactcg 9300
acggccgccg cttcttacag ccttccttct cttctgtttt gcagataacg gggaatggag 9360
accaactgcc gcaaactggt gtcggcgtgt gtgcaattag gtaagaaccc ccttctcctg 9420
cccgggtcat cggacgggag gccgcgccac gtgagggcgg caagagggca ctggccctgc 9480
ggcgaggccc cagcgagggg cgcttccccg aggggccagc ctgggcagga aggaaatcag 9540
aaccaaatcg ccagtggcct ctccctgtgg cggggcggtg gactaggaag cagcggcgcg 9600
ctgtgtaccg aagcccccca gcctactcct cccggctgga accgccggca accggggagg 9660
cgcagaaaga gtacgccatc ctgccccggg ttgccagagg gtccggggga cggggatgcc 9720
gtcagctctt tcctctaact gggtctttgc tctttgtccc tctttctcct ccggcctgcc 9780
tcgcccctcc ccctcctccc gtccccggct cctttcggcc gcggtccggg acgcctctct 9840
ccgcacgtgg ggcgggcgcg cgcgtggccc aggcgtgcag ccggcggccg ttgaatgtct 9900
cttctccaaa gactccgaaa tcaaaaaggt cgagttcacg gactctcctg agagccgaaa 9960
agaggcagcc agcagcaagt tcttcccgcg gcagcatcct ggcgccaatg gtaaggccgg 10020
gagggaagcg caggccgcgc gctgggcacc cgcctccggg actctgggcc tcgggcgaag 10080
cgcaagaagg cgaggccgcc agacctgacg cgcctgctgc ttgaacttag acactccgcc 10140
tctgggtggg acgggaagca gccgtcccag ggacgttaat tcctttcccc aaattatact 10200
gcactcctga gacccaacac ctctctctcc ctctcgctcg ctccctgtgg tctgatcccc 10260
tgcgcacgct ccagcaaacc tcggcgtcta ggctggcgtg gaaaagtggt ccaacagcga 10320
cctctgtcgc tcgttatatc ccgctgcgcg cagcaccacc agggcccact cagtcactca 10380
ggctctcagc cacgccccac ccagacttgt gggtgcgcgg ttcatcgcgg gaggcaagca 10440
agggaaactt gagttggcga aggtttgctt tggctggttg ggggaggggc ggggggccga 10500
caacatccct gaagagctgg agggcagcca ctgtgcttag cagcccaggg tagaatggag 10560
gtttgcccct gtcgacgcga acctgcctga agtactggtt gccaggcctt gggttttggc 10620
gatgtcgttg cttgattggc tggtttcact ctggaggaat cgagggacat tgccagagga 10680
ggtctacagg cttatgtaaa aagttaaaaa gtctccaacc tacgccacag gcctttcctg 10740

aattgaaact tgttctatgg ggcggagggg ggggtgtaag ggatggagga gggaagatgc 10800
ctttcttttc aaatacatgg aaaaaaaccc ctcaaattta ctgttcctct attttcctgg 10860
ctccgtagta aataagtgcc tagccttagg aggctattga cctttgataa tgtgagcaga 10920
taaagccccc cccccccac agccctcggc ccctaacccc ctctttccgg attaaagtgt 10980
aagaatacaa atgtaatatg ggatggaggg gggcgatttg ggaccccggt caaaaaaaca 11040
aaccgtatta ctaagaagaa aacaaaggtc ttgcattgga gtttccccgt gaatctgaga 11100
gaaaatgatc atttgttgaa atgaagcgtc taaagcgatc cagtgcttca cgcccggaca 11160
ctgcactaca ccgccagtcg ccctgctggg ccagttaaac gctcacttgt ccgggattaa 11220
ccccatgggg ttaaatgggg gcaatgcaga gataacgtcg cgtgatttct gtcatttaga 11280
ttgtgttaaa ttcttcttct gtttgataat cggtagtaaa aataaattat tagaccgtag 11340
tatgtttggg atatggttaa aaatcaagag cagcgatgac ttctggggag aatgctttgc 11400
gcgggctcag ccctggctcc ggccagacta gaggagctcc ccaatctcgc ttcgcgcggg 11460
gcgggctcgc agtcgccaag ccgaggctga catttcttat tgtgctggga gccagagaga 11520
cgcaaaatgt ctccttcccc cagtccctac cccaggtttc ctagacatgg ggaatgcatt 11580
ctgaggacag gtggagaagc ctacggtagg atggggtcct cgtaggtgag caggaaacgg 11640
ctaagagcag aggagttctg cttgcgccag tcacaagccg cgcaggcgcc cctggtcgtt 11700
cgcttttgat aactagcaca taaagaacta gaaataatga atgattgctt tcttaaccat 11760
tactttcagg cccgcattgt tttagtgcac gtgaaaggct cttcccctac actcaatatg 11820
tctttttcta ctttttgacc gaaaagaaaa attgctgcct aaacacgttt aatgccatta 11880
attaagaaaa ggcatgtaat gggaagaaat gctgaaaatc ttgatttaat tggcttttaa 11940
ggaactagta gacgacaaaa aaaaatcaca cgagtgggca aagctatagc actgctgaag 12000
gatagagcac ctatccttcc ctgattttaa gttaacttat ggaatatcca aagtcctggc 12060
cacagcctgc ttgtaaaaca aaaggattta tttcttgtgt ttttttaaag tttttctttg 12120
cttttaaaga gaaaaaaagt tcacaatgac atatgacttc ttcaaaaggc cgtgatagtc 12180
tattacgcta ccctctccgc ctctgccccc aacgccgccc aaaaatacca tgtcctcgtt 12240
aaagactaaa cgttctgtat aggcagagtc cacctctaag cagtccaggc tctgccttcc 12300
ttccctagtg agtcccattc tccttggtat ccattgggcg gatgcccagc ctggatagaa 12360
ccccgaaacg gggggtagcac gagagcgact ggagacccct aaaagccaga ggtttgagag 12420
agggtggacg cagctagcag aagatggtgt agaagccagc tgagaacgac cccctagagc 12480
aaagagacct ttctttggct tttcttgctt tgggggtcct gaaaggaact cataaaatgg 12540
tcttcaccct caggaggagg acggactgac cctcctctgt cactggctta aaaagtttca 12600

gggcggcggc tctgggttcc gtgctgaaat cggactgcac tgcagcccct ttggacctga 12660
cgcctggctt tgcgtcccga caaggggcgg gtacttcccc cggcctcccc caggaacgca 12720
ttaattgtta aatagctttg gcccagtgga tgggctgaaa gtgttcgacc caagtcgctg 12780
gtgtgcacag acttcctccc tctgggaggt gggctccatg gcccgttgtg gcacccccag 12840
ccgcgacaca caccttcaca cgcggcagca gctcggtccc aaccttcttc gaaggacctg 12900
ggttaaccct ggcggccttg gcggccgcag acccctctcc gccgccccgc ccccgcgcct 12960
ctcattcaat cagcgaatgt ttgcggagca tatactacgt ggactcctaa tgtacccccct 13020
gaaagcaaat aatacagttt tcctcgccgt catgaaggga ttttaatcct aacatggaca 13080
ccagcgagat cagcccagac cgtctctagc aaaacgcaaa atggtggtgc gtggggtggt 13140
gaccaaggtc ctgagccttg tcagaaagaa ggggatgtgt agagaaaggt ggagaactct 13200
agctgtggct agcgcggaag ggacaggtgc ttgccgaagg gggcatgagg cttgaggaaa 13260
aagcaacgaa ataggggtaa ggagagtttt ccattttctt tcctccgccc gacctccgcc 13320
atcccattct cccctcccct cccccacccc ccgcgccaat caaatctgca gctcacttga 13380
aaggtgcccc gccgcgttgt ggtttttcac ccccagggga aattgtacca gttgtccgaa 13440
agtagtcagt ccctgcggat ccctgcccgc aaaagtggtc ttcacaggtc gcgttcctcg 13500
ctgctgactc ggtacacaaa gtttcttaag gctggttcgg ctgttattcc tcaccgcccg 13560
ctgctaatat atgcagcagt tgttagagcg gctcggggga aaaggaaatg tataacgaaa 13620
gcttattcgt gagcaggaat atactaatgg aacaatctga tgtcttctta atcctatgta 13680
aaaagctttg tcgtcttcct aatattgact gaatgggtaa ttaatggctc ttcatctagg 13740
cgaataccct gtaatccaag ataggcaaaa gacaacaagc ccaaggtaga agacaaaagg 13800
cccaactgca gcggcgtttg cccgcctcct accccccagg gtctctgact aggaaagttt 13860
tctctcagag gagaaaaagg caggagtggg agaatatata cctatcattt cggggctaga 13920
cttcaccgca gcacctgacc acccagctca gttttctgtt actctgtctt tccacctcca 13980
gtccttctcc ctgcattttt ttttcttctt aactcctcta ggatgacctc ctaccaccac 14040
cgccattatg gtcctaataa cctttccccc taaaccccac atctttcact tcagcaacca 14100
atgaggctgt tttctgatcc aggaggagat tcttttcttt tagaacccaa tgcgtagagt 14160
ctttgagaac taaagtagtt ggtaggggag gaagaaatta atagaaaggg agagagcata 14220
cagaattgtg tgtgtatgtt aaagagcgac caggaatgac agagtcaact tctttgtgag 14280
gatctgacgg gaagagtgtc caagactcta ccagcatgtt tttaacaggc tgacatttta 14340
atttaaacct ttagaagtaa tatattactt gggttactac aatgaggtgg gttccttttt 14400
ttttgtcagc tgacagcttt taaaatatta tttcgctagg gaaataaaag cttcatctca 14460
gattataggt gggtattttt ggatttaggt gatttatggt caccatgaca actaatgctg 14520

aatgttagct accagcatgt ctgggagaga gaaaacagaa agaagggaga gcaaaagaaa 14580
tagaaaaggg agatggacat aagctggaga gggaagaaaa gagaaaaaga ggaagacaga 14640
tgagtgttta atccaactgt tgtttaaaaa agtggcgggg gggtgggact tcatttagcc 14700
ccttgccact tccttctttc ctgacctgga tatctatgct caatttcata ttccaccctt 14760
ccttcccctt ctccaaacac atgtgctata ccattctcct tattttactt agttcggcaa 14820
gtagttgctt tctggagact cagtgacacc taggaaaact gtggcagtaa catgcaaatg 14880
tgaggaagca ttaacagcat gttcgttgag tgattttagc aaatgccccc tcctctaatc 14940
tctttctctt ctcttcccca ggccactgtg aggtggtaac tcccactgcc atctgaacac 15000
tgttcccccca ggtagttacc ccaaatctca aatggttgag cagctagagc tgtgggttgg 15060
aaaaatgggt accatttgca gggacccaga gagggtggct gttgctcaat atatctacag 15120
acttccaatt cagaaaataa tcttcccctt ttgacaagcc agagcttttt aaattccatt 15180
taggaaatgg ggaaaaggac agctacagtg aagcttttaa tttctgggtt atttggttcc 15240
atagctatga ggggtggtgg gtagtggact gttttcagct tggtttgtat gcagagaaaa 15300
gccagatatt ggaggggggtg gggcacttct cgggcaggat gcaaggtctc catctgacct 15360
ctgcgcctta ccaggagctc acacactcac gcccatcacg tggctccaag ctgagtccag 15420
gcgggcctcg tccttgagct agcttgggca gggcaggact cccacccgtc taaggcctaa 15480
cagctcaggg agatgtctaa ttaagccatc ttctgggtga actctgaaga cagactcttt 15540
ccaaaagtca gagatcactt ggttgagtcc caggccagat tgatacggag agtttggcgg 15600
cacagcccaa ccgctcactg ccatggccag agggactttg cacaactaac actgaagagt 15660
gtgaaattaa ataagacttt aagactggta atcggtggca aataccagca caaaacacgg 15720
ctgaccccat gtcacacatt tccctcctct agggtctttc tttgaaagaa taagcaagaa 15780
accccaatcg agacaacccc tgatgtctcc cagactcaaa acctcacgcc ggcactgggc 15840
tttccttctt tgccccacgt gagccatgca gcacctctag tttccctacc aggatcccac 15900
caatgttctc cgtattggaa atttctgtgt tagaggctga actcatagta atttctaaaa 15960
ccaattaaga agaacttagc cagaggtcac agtaatgctg gaatcacaaa atgcataaga 16020
tttattttct tctggcccct ttctcatcca tgctgcctat gcctgtgcac ccacaagtct 16080
tatgtacatt aaatctccaa aatcaaccac caccatgcca cagagttttc actggacagt 16140
gtttcttagt tcccaccaca tacctccttt tccccatgca gacttatcat gttggtgtcc 16200
tgccacacag gggcctgag aagaatgtca cccaatcgcc gctgctgtga gtgtgtaaag 16260
tgattaggag attaggagaa tgttgaaact cctgctggaa aaatgcaaag aaaatcctca 16320
ctttgagtca gttgtttaca gagccagtgt gtgtgtgcgt gtgtgtgtct gtaatataaa 16380

atggatgtga atatatatat acaaatagat atggttttgc ttttacttta atctgaatta 16440
tctagataat tgtctttatt caccacttga cttcaatggg tccacacaaa ttaggacacc 16500
ttatcttttt aggcatctag gctgctgctg atccccagtg cttccaacat ctcgcacacg 16560
ttggcactat gaggagcagt cacgtgccct tgggtttttc aaccactttg gaggctgatt 16620
gaggtccttc atacatgtat atttgtcgtg atgaaagttc catcggtaga gtggagccac 16680
cagagctttt atcaaaattc tgtgggttta tgagagatgg gtttagaaat ctatatggct 16740
ctgtggggct tctcggcttt ctaaaataag gcattaacac ccaagcttcc aaaaatattt 16800
gcagctctgg ggtttgaatc ttgaaaaaca aggagtgagg ggctgtgtat actaactaca 16860
gtggagattt ttttcattct ttaatgtgat ggagtccctt catgaaatga agctttaagg 16920
ggcatggtat tgtggggacc acagctattc tgaggtttaa aagaagaaac tggaatatga 16980
ttagtaaaca cattcagcag aaaagagctg gattcttatt gacttagtta taggtcatcg 17040
gctggcagtg caatgggagg aaatatttac tttacacata tactttatga tcttggggga 17100
attagaggaa attcaataag aaaacggcta gaaacattta aaacccttat ttaaaagact 17160
taagcaaatt agagtcttat cagattaaaa accactacaa atgtaagagc attgtcttca 17220
gtgaaacgct gtggggtctg agaaggagat tctccgccaa atctccggga taaaatgcgt 17280
catttaagca ccagataatg agcagaatgt aaattaattt aaccttcttt accaacaggc 17340
tgctagtgta atgtgtataa tttagtgata agattgcagg acctaatata gctggatgta 17400
tgagcctcag ctaatgcaga cctgtcacat gaggatgtgt tttactctga gcaggtgtct 17460
gtatgtgtgg aatggggtaa agtggaataa aaggttaaaa gcagaaatgc tgatttaaag 17520
cttactatga agaaattcct cccttgcagc taaattattc ttaaagtggg atgatactgg 17580
tgaagaaaga ctgaaaaaca attctcatgt gcgtctttgg actgcaagtt taaaatgggg 17640
aggagttgca gatagggttt gggggtggtc agggcaaagg agagacacat aagttgcaaa 17700
tatatttgta gtctgcttca tccactttgt tccacatcga ataagtttcc caatcttgtg 17760
aacaaggaca aggagggagt gttttaaaga tacttcatgc tggcattgca aatcattgac 17820
tgtaatgtca aacaaataca cattcagaga tgataacact aactccatag taaaacaatc 17880
gcccatgcag aaacccagag gagattagtt tgtcctctcc agctgaccta tgctggggga 17940
caaaaggact ttcaaaaatt attttgaata tgtttggatt tctttcttta atttctttgg 18000
aaattaaatt tgcttggaaa cagtgctata aagagttgat gtctccaaag gtgatttttt 18060
ttgttttata taaataaggt tttgcttttg ctagttgagc gcagttctag gcttttcgcc 18120
cttagctcac acacacccct tctgcctgct tggactttaa tggctcaaga cagccttgag 18180
ctcactggga aaagaaaatg actgttaaaa attatccttg aaattggtta tttggcaaca 18240
ttcttaattg tatggaaatt cattaaggca tatttcatat ataattagct caaggttgtt 18300

gattctacag gctttatgga tttaaatctg attgataata aagtaaacaa gagagtcgaa 18360
tttaaagcgt ggctctctcg ggttaggacg agcttaatac agtgtacaag gaatttgaaa 18420
gatctaggat atgtgtctta atcaacgtta agtagaatgg ataagctttc agcattttga 18480
aaacgctggg ttagggtttc tcttctattg tgtgttttct gtctggggac taataagcat 18540
cacagagaac gtgatctgag gcgacttttt attcttgtat aaatccagag tgaaccacca 18600
aacagttgtt cgtttaaagt caaggtaatt ttcttttgac gggtccattt gcttctcgat 18660
ttctaattta ttagcctgcc ttttcagggc tctgtcttct ttgcaattaa agcttcttca 18720
gattagcgca gcattcactt gacaggctgt ttggaaaatt taagatcgga gaggtgattt 18780
gttgctgttt ttcaaatttt ctagttttaa gtaacgtgtc tcctttttat atggggtggg 18840
ggattggaaa tggatgtagt gagacacaaa gagtgggtgt cttgttgatc cttgtacctt 18900
tctcttcttg accattccac tctcttctcc caagccttcg actcctagcc tcatctcttc 18960
acctttgggt tcgtactaaa agccggatcg ccttgggctg ggcaggagct gaattcccgg 19020
gagcttgcct gtgtagaccc agtgcgcacg gcgaggcagt agcccggccc cgcactgctg 19080
ataggtgcag gcaggacagt ccctccaccg cggctcgggg cgtcctgatt ggtgcggagc 19140
cacgtcagtc gcacccggag aagggtctgg gaggaggcgg aggcggagag ggctggggag 19200
ggccgcggcg gagtgacgtc tcggcaccag gaagcccgcc tctggtttta agatgttagg 19260
ccaacaggga agcgcggagc cgcagatctg gtccgtcgct cgcctgggtg cctggagctg 19320
agctgcggca aggcccggct cctgttcgac cgcccgaggg gtgtgcgtgt gcgcgttgcg 19380
gagggtgcgc tcagagggcc gcgtcgtggc tgcagcggct gctgccgccg caggggatct 19440
aatatcacct acctgtccct gtcactcttg acacttctct gtcagggctg ccgcgtgggg 19500
gggggcgggg cagagcgcgg tcggcgttag ctttccttat tggaggggtt cttgggggag 19560
ggagggagag aagaaggggg tctttgccca ctcttgtttc gctttggagc ttggaagcct 19620
gctccctaaa gacgctctga gtggtgccct tctgcccaca tcccatgtct tcgtttgccc 19680
gctgactttc cgtctccgga ctttttcgct tgagccttcc ggaggagacg gggcagctt 19740
ggcttgagaa ctcggcgggg gttgcgtccc ctggctctcc ccgcagcggg gaaactccgc 19800
gcctagagcg cgacccggag cgggcagcgg cggctacggg ggctcggcgg ggcagtagcc 19860
aaggactagt agagcgtcgc gctccctcgt ccatgaactg catgaaaggc ccgcttcact 19920
tggagcaccg agcagcgggg accaagctgt cggccgtctc ctcatcttcc tgtcaccatc 19980
cccagccgtt agccatggct tcggttctgg ctcccggtca gccccggtcg ctggactcct 20040
ccaagcacag gctggaggtg cacaccatct ccgacacctc cagcccggag gccgcaggta 20100
aggcgccgcg ccgccctgca gacattcccg ctcagctgct ctgcgccacc cgctccctct 20160

cgccccaagg aagtcagccc ctccgggggg aggcgtggtg ggagtggtcg ttcgcctggc 20220

tccccgcaga acttccggga gccggaattt tgactacccc gcatcccttt agttctccct 20280

cgaccggccc ggctcctggg gcgctaaggg cgcgagcaat tctgccgccc tctctattcg 20340

taccctggcc tcccttctgt ttcctgggtc acaaaaatcc cagcatcttg attcgaggac 20400

cttcagaggc cgccgacctc tgtccctgtt ttcctctcgg ctttcagctc ccgaggagct 20460

ccactcgtta ggaaattgcc tgaaaccact cagaaatgcc cttcgcgaag aggcattttt 20520

tttttttttt tgggaaaggg ccggcgaact tcggtgccca accgaatccc cacatctttt 20580

cctagccttc ccaaaccgca tggaaatctg agctttctgc gagggggagg ggggtctgta 20640

aaccacgcgc gtgtgcgcgt cccaggagat ttggtgtgtc tgcgcagagg tgtataaata 20700

tacttgaaag cacaggctat aaaagtgaat gtgccgctgc agtgagataa acatgtaaat 20760

aaaacgtgcg gcgctggggg aggggaggaa atggggcgcg gacacccaca cttgcgcctg 20820

cacaccccac aggcgcagcg ctcctcgcgg cccggagccg ccgcgcgcac cctcctccgg 20880

cgccaggcag cccagctctt ccacggcttc tgccgccggt ccagttggcg tccgcgttgc 20940

aggtgggcat gctgacggga aagtgtgtgt gtttcgtttt cagagaaaga taaaagccag 21000

caggggaaga atgaggacgt gggcgccgag gacccgtcta agaagaagcg gcaaaggcgg 21060

cagcggactc actttaccag ccagcagctc caggagctgg aggccacttt ccagaggaac 21120

cgctacccgg acatgtccac acgcgaagaa atcgctgtgt ggaccaacct tacggaagcc 21180

cgagtccggg taggagccag cacggagtct gggagggatg gggggaggat gttgtggagg 21240

tacaggccaa gtagaccagg agagaatgtg gaaggcagcg ccgcctggga gggcgccggt 21300

ggggcgcagc tttgcaaagg cagaaggcct cgcggcggcc tggttgcgag attacagttc 21360

cctctccgag gccgacagga ctgccgccct ggctcaggct cccagagcgg caccggctca 21420

ctgccccgcc atcccgcgat ctcacgagct gggctgcatg ggcaatcccc tgcacaggac 21480

attgtgttcc tggcttgcag ttgccagagc agagctaata aaatccctac caggccaaga 21540

gccgcgaaca ggctccaacc tgtgagcctt taacaaggaa aacccgccag agacacggaa 21600

gagttggccc tccctgggaa acctttgtcc cggccctggc ccagcttttt ccctcctggg 21660

ctcgcgcttc ttacaccttc tttacggttg tttcggccat tcaggtctct cccacacacc 21720

ctatttccta gttttgtgat ctccgggagc aaagttttaa tacacaacta ctagtcctct 21780

tagaaggaga aagaaaaaaa gaagaaagac ttttctgctt ggtttattta tcttctctca 21840

ggagttgaac tctggaaatt gaaactcaca ccccctcttc taaattataa tcatagtttt 21900

gtaaaaaggg cttaccttaa ctttgtagca aatctgtact ttatggattg gcaaaaatga 21960

gctcaaataa ataacccaat agcaacgtcc tggtttatgc tggtcggtgg aagattccaa 22020

atttgttagg attctggaag cagaaaacag aatcaagcaa atcaagcggc atccagaggc 22080

tttgctgtta aaaaaaaaaa attaagtgct ctgggtagaa aaaataaagc ccccggttag 22140
agcagagcaa acaaaaagaa gaaaacaacg ataaaaagaa taaagaccaa aatgctctcc 22200
caaatcagag ggaatgaaga cactctctgg gtggcatttg tgcaaggcat gaggctatgc 22260
tggtggataa aaggccggga agaagttgaa aatggtttta gtttaactgt ccagagccag 22320
agctgggtcc tgggcggcgt ggttttgagc aaggtcagtc tttcattagc tctcttgcac 22380
atcaagggaa cgggcctctc acgtactctt ctcgcctgag caaagtttag atggcctagg 22440
gtagaaatgg caagtaatta aagacagagt ctatgggttt tctgggatcc ttcgaaaacg 22500
ccctcccacc ccgcccgcta ttccgcagct ccaccctagt gctttgtagc cgcggcgctg 22560
ggctctcctt gcagctgcct ctccttccag ggcggctgct tgtcgagcca agtgggagtg 22620
aggcgtgctt tttatagcag tcgggtgcaa agaggaaggg ggataaaaag gaaatcaaga 22680
atgaaaggaa aaagagaaaa agcggattac acggctgggc ccggcggaga tgtgtaatgt 22740
gaaacatcac tggtgtcagc tcggatatct caggccaggc ctctctccaa tacacaaaag 22800
ccgccgtctg gggcgacagg gaggccccgat gtggattggg atcggggttg cggctgggcc 22860
accggacacg ggtggaagcc ggccggcctg ggtggccgcc tgcaaagcca aacgacccgg 22920
ctgggcctgg cgcgcggaca ggcctgtggt gggcttaggg taaagaagag gcagagcgaa 22980
agaaggggga atctccaaaa ctaccctttc cgggttcccg gagtttaata tgccaagctc 23040
ctggagttaa cgagctgacg aagaggtggt cttttgctct ttatttggtt gttttgctag 23100
gcgagaaaga gtgttggcgg cctagtccct gttaagggag cacgtaccag gggtggggg 23160
acgacagtgg aggtcaggga aggaagggag gaattgcgtg ggagaaagag cgatcctcta 23220
gtgcccttcc agccccttct cctcatccgt gggtctgtgg ctttggaatg gaagcaagtt 23280
tgcaaggtgc cccgggaagg gttggaaaag cctgctgccc cgcgtttgtt ttacattaag 23340
tgtttttgga cctggagaaa cgcctggctg agtgatcaaa ccgtccgcag gtctccatgc 23400
gttcggctga ggtttgtggc gtagctccga gtcccagctc gcaggccaga gcagaccagg 23460
tctcctgcgc ttggtggaga cccgggccag taactgaaag ctggccctgg tatcttggtg 23520
tgcagggcgg tgcagtgaag cgaggccagg gtgtgtgagt gcgctagcgt gtgtgtcggg 23580
ggaaggcggg ggttggcctc cgatggaagt tttagtaatc tgcactgtgg catctgtttg 23640
ctcccttgcc ccaaccgccc ccaggtttgg ttcaagaatc gtcgggccaa atggagaaag 23700
agggagcgca accagcaggc cgagctatgc aagaatggct tcgggccgca gttcaatggg 23760
ctcatgcagc cctacgacga catgtaccca ggctattcct acaacaactg ggccgccaag 23820
ggccttacat ccgcctccct atccaccaag agcttcccct tcttcaactc tatgaacgtc 23880
aaccccctgt catcacagag catgttttcc ccacccaact ctatctcgtc catgagcatg 23940

tcgtccagca tggtgccctc agcagtgaca ggcgtcccgg gctccagtct caacagcctg 24000
aataacttga acaacctgag tagcccgtcg ctgaattccg cggtgccgac gcctgcctgt 24060
ccttacgcgc cgccgactcc tccgtatgtt tatagggaca cgtgtaactc gagcctggcc 24120
agcctgagac tgaaagcaaa gcagcactcc agcttcggct acgccagcgt gcagaacccg 24180
gcctccaacc tgagtgcttg ccagtatgca gtggaccggc ccgtgtgagc cgcacccaca 24240
gcgccgggat cctaggacct tgccggatgg ggcaactccg cccttgaaag actgggaatt 24300
atgctagaag gtcgtgggca ctaaagaaag ggagagaaag agaagctata tagagaaaag 24360
gaaaccactg aatcaaagag agagctcctt tgatttcaaa gggatgtcct cagtgtctga 24420
catctttcac tacaagtatt tctaacagtt gcaaggacac atacacaaac aaatgtttga 24480
ctggatatga cattttaaca ttactataag cttgttattt tttaagttta gcattgttaa 24540
catttaaatg actgaaagga tgtatatata tcgaaatgtc aaattaattt tataaaagca 24600
gttgttagta atatcacaac agtgttttta aaggttaggc tttaaaataa agcatgttat 24660
acagaagcga ttaggatttt tcgcttgcga gcaagggagt gtatatacta aatgccacac 24720
tgtatgtttc taacatatta ttattattat aaaaaatgtg tgaatatcag ttttagaata 24780
gtttctctgg tggatgcaat gatgtttctg aaactgctat gtacaaccta ccctgtgtat 24840
aacatttcgt acaatattat tgttttactt ttcagcaaat atgaaacaaa tgtgttttat 24900
ttcatgggag taaaatatac tgcatacaaa ttggtctgga ttctttctcc cctcctctgt 24960
cactaacttg gccaggacat ctcagtcact gcttcctaaa caaaccagtt ccctctgcct 25020
gcctagttaa acatacaagg cagcagtcct tatttaaatt tggtagaaat aaatgatagc 25080
cattcatcag aaactaaaaa gaaaaaaaaa ggcattcccg gggggaaaa gggctacaaa 25140
atctaatttt gtctctccaa tttttctttg gcttaaacct agaggattcc attatggcta 25200
gcaaataata tgaaaaagaa aaaagaagaa agaaatttag caagtccatc agcttaaaat 25260
gactctcaag tttactcctt tacggggaaa cctacacctc tagcaaattg ttctggagaa 25320
atatttgtgc atgtatacat gtatagttta tatgcatttc tctcaggagg aatacatcta 25380
taataaattt acagggaaac atctccagtt caaaatattt aggcttccac gtttatcttc 25440
aggcttaagt agagagatcc ttcatgttat attgcattac tatttccaaa tcctttggag 25500
acattaaaag aaacaaagat gatttctaat aactacagcc cttcagtttc tcaaagaact 25560
caggggttga gaggttagag tggagtttcc tgagtcttgt cgagcaatat gtagttgagg 25620
caaaggtcat gctcccggtg ttttgtttta aataatattg acccattaat tctaaacctg 25680
cttgttcctg aaattataca ggattatagt ttgcaaactg caggacaatg aagcaaatca 25740
agatgaatta cagccctggc cctccctgcc atcctctgac atctaaacag ggaatgagtt 25800
cggtgtgagt gtttaaatga actttaagca cccgatcctt ctttatccgc gattttcagc 25860

tttaaaaaaa tgtgaaattt gatttcataa caaatagaaa caaataccac ttagtcccag 25920

agaattcatc ctcatggcgc taggagggtc gttgtggagg tgggggaggg gatgtgctga 25980

gatcttttgt tatgcttgtc aacccccgc acaaccaaag tgggcgagaa caaacaccac 26040

gctggggaac ttagagcaaa aagtaaccgc cgattttctg gagccgacaa tatcattgtt 26100

ttttcgcctt agttgctttc atctgaatga aactttacct agaagctttt ggagctcgaa 26160

cactgagtct ttgttctgca agaaactgct gctgccactt aaagagatcg cagataatgt 26220

ccccgattta agatcagtgc tcaacgcaca ctttctttct ttttaaagct ctgcctccga 26280

cttgcggagg gatcacgcag cagtgggggg cagataaaag ccctttggac gagggtcacc 26340

tcccaccatg ttgttcactt gagagcagtg gagaggaaaa cggtccccac gggcggactt 26400

tggcttctgg agccgcaggg cccagcggtc ccggcctctc cgcctctcag cctggtaggg 26460

ggcggggagg gccaaagggc ggaggggaag gaaggagcta gaagagggga tttggggatg 26520

ggggctggaa gcgctaacga gacctgcccg gaggatttag gtctcctgca ggttggtgag 26580

tgatttggga gctctgggta aaagaggtgt acctcggcct agtttggttg ctgaactaga 26640

acaacgcgag gacatcaatc aatctgcagg aaacaaaaaa tggaagtcga ggtttaggaa 26700

gagttgtcac ttccccgact tgagtggtgc aggctggggg cggagacttg ggacctaaga 26760

gaggccattt ctctcacctc agctcccttc ccttggactt ccaaaaggaa taattttact 26820

ctctcctccg ttctccccac aactctcatc cccgctagcc cgcaggccgc ctgcccttt 26880

ccgcacctcc ttttcccatc cagcgagaga aacctagctc ccagagccgg 26930

<210> 2
<211> 22020
<212> DNA
<213> Homo sapiens

<400> 2
ctcccttccc tctccctgtg tctctccgcg gatctctgaa tctttctgtc tttggttttc 60

tcattctctt ccaacttttc catgagattg cctatcctcg ccaccagctg aaggcaaggc 120

cgttctgcta cgagcgcctc ttaatctcta caaaatgaaa agaaaaaaag ggaggattat 180

tagcccatta ctcagaggaa tggggaggct gcaaaaatcg tcgatgggca gaggtgaaga 240

tgtctttctc ggactgcact ttccggtgtc ctgtaactag agttcagttg tgggacttgt 300

tgaagaaatt tgattttctt gcctcggcga gatttcaaaa accagaaata gaaattctca 360

gagtcagaga ggaaatacaa ttaaacagca cgtgggcatt ttccccctca tttctctccc 420

cttaaataac actgctttga gtttccactg ggtaaagaga gaaagtttga gttttcacgg 480

atgttacgtg gaggttagaa atggcttaaa atgtagatct ctaatcagtt ttcttcgtgg 540

ctgaagaggc taacccttc cataaaatga gtccatctgt cgactgttag ctatttcaaa 600

```
gtgaagggat ttagcactca aaacaaattg agcaagtttg tttgcctgtt tttactgcta      660
actcaaatga attcaaaaca cggagtaatt caagaaaaca cataacatgt tccagacagc      720
ccccaaaagt agggaaagcc cagcacctat atagtgacta gggttagttt taagcgccaa      780
gcttttttaa acgtatctat tttatgcaca ttctcccgag tcactatata tttctaaaat      840
tgcgagtatt ggtatattga tttaggaaga gcaatacaac ttttagaggg aactttattc      900
tcaattaggg accaaagaga tgtcttttta atagcgggcc tgagttttgc tctcaagcag      960
gaattaatat tggtgggaaa atccgaatcc aggagcaatg gctgtgttcc ggcactttcc     1020
aaaaacatac attaacagga tgcccttgag attgaaaaaa cattgtccca tatgcctggc     1080
agaagccttc acacctggtc ctccaggcga attatattta tagtccttcc actcagaggc     1140
aggacagagc caaaatattc tgctcactac caaaatacac atttttgctc aagtcaagaa     1200
atcagaaaat caggggttcag aagtaaggca cacttttcga gtgagaatat gcccctgtaa     1260
tttcacatac tctttgcttt gcaggagcaa atgtggactt gagggaaact ctctccccca     1320
cccccacttc tatcccgtgc aatgtaatac catcctcgtc aggaacctga acctcgtcat     1380
tttaagaaat gagatatccg tgactcaggg tgaacttgtt gaatgtaggt acagcagagg     1440
aaattctaga ctctatgagt gtttgagcct tgtccagtgc aaacctttcg tgaacactgg     1500
gtcagtgcgt ggccgtgccc acctgtgcgc agacactctc agcatgcctg gtccacccgc     1560
cttgacatcg ggcgcggtgt cccagctaag ctgggcccag cgtcccggcc ttccccagct     1620
gacaagcgta ggtcgttcgc tcccggcttg tggccctccc accctctccc actagctcac     1680
tccattgttg tagattctct ctctcactca tcctctccca tccccaccgc gcccacctcc     1740
actcccgccc tctaccggtc tctcactttc ctccctccgc agtccctctt tgctgtgacc     1800
tctttcctca actctgcagg cctgaaagaa ggtcacacac gcacgctcac acccacactc     1860
cacacgcctc gtcccaaaca accccatgaa cattgtcctt tgttccgtct cttgggccac     1920
tttccctgtc gttcctccca gcccgtcctg atttgctccc caaaagtacg tttctgtctc     1980
cccgctgccc tggcgctccc cctttgattt attagggctg ccgggttggc gcagattgct     2040
ttttcttctc ttccatccca tcctcccttc tggtcctcct ttccacagtg ggagtccgtg     2100
ctcctgctcc tcggttggct cctaagtgcc ccgccaggtc ccctctcctt tcgctctccc     2160
ggctccggct cccgactctt cggcccgctg gcatctgctt ccctcccctg cctcgtttct     2220
cgtcgcccct gctcgctccc cccggcgctc gcccgggcgc tgtgctcgct cctggatcgc     2280
cagccgcgca gccgggctcg gccggccgcc cgcgcgccac tgtgcagtgg agtttggtgg     2340
aatctctgct gacgtcacgt cactccccac acggagtagg agcagaggga agagagaggg     2400
atgagaggga gggagaggag agagagtgcg agaccgagcg agaaagctgg agaggagcag     2460
```

aaagaaactg ccagtggcgg ctagatttcg gaggccccag tgcacccgtg gactccttcg 2520
gaacttggca ccctcaggag ccctgcagtc ctctcaggcc cggctttcgg gcgcttgccg 2580
tgcagccgga ggctcggctc gctggaaatc gccccgggaa gcagtgggac gcggagacag 2640
cagctctctc ccggtagccg gtaagtggag gccatctatc ccgcagggat gtgagataat 2700
gcgagtctgg aaatttgttc cacttcggag aatcttcacc gtaggtgatt tgtggctttt 2760
ggggctaagt ttcgcccaag gtaacgcagt cggcaaacag accttgcaaa gccctgttcc 2820
tttcgtcccc cgccacagac actaacaatc tacagggtgc tgaagtcgag agggaagcca 2880
gaccgtggct ggcatttaaa acgaggtatc ttcccttaaa tctcggtgcc aacactgcag 2940
gaacaaatcc tcgggccaag gattagcatt ctcaagataa agggctgggt acaaagtttc 3000
agctactgga agattagccc ccttcccatt gttatccatt gggaaaaaaa agaaaagaaa 3060
aagattccat cttaactggc agttagtgac ctctcaggcc caagcgaatt acctgggagc 3120
caggcctgga tgccaagctc tcaccatttc tttggattgt aactccttta aattgatcac 3180
cagtcaactc caatctggca cttcaggaga tacactttaa atggatgcag agaattattt 3240
tccagctgga gattaagaaa aaaattttcg attctaaacc tccgaaatat gttcctcttt 3300
tccagtttaa ccactttact ttcttaagca atttagtaat caaactatca taaggtggtg 3360
tgattttttt ttactctttt gtgtgagtat tgtcttacta aactaaacgg aaaaaacttt 3420
taccattata aatgtaaata tcagaattca tacattctaa aatattttta tgaaaaatta 3480
atctgattta aagaaatttc cttgcatttg ttttagtcta tcaatcaaaa ctaaagatgc 3540
ttttatcaca caaaatatca ttttggcaga aatccatcta aaattcaaat accaataata 3600
tcaagaaaac aaagcacata agcaaaataa attgaagatt tttgttgatg taacatgagc 3660
atacaacatt tcaataacca aactttccct aaaaaattaa atagccactt catttgtgga 3720
atgttttact ttaactcagc aaaattacac ttaaattatt taggtgcttt gttcccttaa 3780
gttaagcgtg tttgtcttca aatgttccct aaagcactta tattaattgg ttgtaaagaa 3840
cgcatacaca tggtaaaata cagaactgaa ctgagcagta ttttaatttc ccttaaataa 3900
ttacttacta caaattaatt tactggctaa tttcacaatt tagttcattt aaaacacatg 3960
ttcctgtgct gtttattttt aaactttcca ttaaagattt tgttatgggg taacaaagtg 4020
tatgaaaagg ggggaaatgt gaaaggatct gggattattc gaactgtatt tttcctgcac 4080
tttcagtctt gcggtagtca tcagaaatta ttttttagca aattgtttta tttcttaggg 4140
cttgcctgcc tgctttgcca tggttcctca tcctccatta gccgtgtagt gctttttgtg 4200
tgctcacaat ataaaaccca agttggccaa aacaagagtc ccttggcata tacattccaa 4260
ctagaacatg aactttgggg gtgagaacta cctcccatca ggaaaagtct cccatctcaa 4320
tttgtgagat tagccattga agccagttcc gaagtctggc agccaaattt ctcacagaag 4380

acttgtcttg atagggcaag tttaaggatc agcaggcggg aattggaggt ctctttttaa 4440
aaaattatct tccccagtta tttagactca gttcttctag taggcctggt cattaaatga 4500
agcataaaaa tgcaagtctc aaggctcatt ttgactgcaa aataaatctc caagtcacaa 4560
ggacatgtag gagtgagcta aggaacacgc cttgaccttc ttttcagtcc ttagagtgga 4620
gctctatgag ttcttgaaga tttgttttgt attgctttgt ttggtcttca gcactgaagc 4680
acggggaagt gggggggaagc atgtgtaata attgactgac tttacaccaa gcaacgcaat 4740
ctttttcttc tgtatatttc attctttaaa aaaaataaat aaataaaaac tatttgcagt 4800
taccatctgc agtgctccgg ctaccagcta ataatgcagc cagttcagac atataaaaaa 4860
aaaagattat cgaaatgatg atgacatgca aatttcctcc gaaattatca taagtaaaca 4920
tttgaagtct ggactaataa aatcccatct gtgttacttc atatcgagtt agtagaaagc 4980
tgtgataatg aattttgtaa tatctcacga acagacatct caatcaggga ctaatcctgt 5040
gattttactg cagaatcact aaatctggag ccgccaaact gctacttctg ggcccacggg 5100
cccacaagga tcgaatcggc agagtccccg cccgcgttct cgctagcggg tgggggaacc 5160
gcctggccgt ccccaccctg gatccccacg ccacagcgcc gggcagcccc tcctgtaggc 5220
agcgaccttg gccagaggct ccccagggcc cagctccctt caggagaggc cgagacgcag 5280
ggaaacggta ctcaggccag aggcaggccc gcagctccct gccccgcctc tgtgcctccg 5340
ccaacccgac aacgcttgct cccaccccga tccccgcacc cgcgcgaagt gggccctccg 5400
gtcgtcggcg taccctggtt agcgtggaga gaggcaggcg ctgagatcga aggggcctag 5460
ggagccctgg accttctttt cttgtcttta aagcaaccgc ggctcttcta cccacccggt 5520
ggagcccctc gagacccacc tctcccggct tgcctgtggc agagaagggg gagcgcgtta 5580
aatgcttggc tcgctgcgtt gtggttgaaa acgtgaaaaa gatttggctc gcccgggaga 5640
gaaaggggga gaactgggta gcagttatac cagagctatt tctccgtcct tggcgggcag 5700
taaaccctcc aagaacgttt gccctgctct cctcagtctc gctcagtcca cccagtgtct 5760
ctcctctgcg atcttaaatc atactttagg gtaattattt gtagtaagta aataaatggc 5820
cgggttagta tctctaggag aaagtgtggc taaatatgga aaagtggctc ctgatggatg 5880
agaggcccga actcagctcg ctcctgaaac accctaggcc aagagcccgt tcgtttcaga 5940
attacagaaa accgagggaa actgctgtct aggacagggg cacgttggcg ctgatgttct 6000
acaaatgttt accgagctcc aactaatgga caagcactga agggtggtct ttgcatacag 6060
cttcccaaag agaaaagtcc tctccaccca cccactcccg ccgccattgc gttcagatga 6120
gttcttaacc ccggcaccga gattcttgaa agtaggtcca cagcctcccc agcacactgt 6180
ggctttatag tcctctaacc tctgggcact tttgcggcaa ctctggaggg agatcccctc 6240

ttgataaata aatgtcctgg gcccgaggct aggctggaga tgctgctgca cagccagagg 6300
ctgtcaggtc ggaaaaacac gcctgaagcc tagcacacag taggcgccta acagctagtg 6360
taacgtagtc tcatctgagc cctgctcact cgacggccgc cgcttcttac agccttcctt 6420
ctcttctgtt ttgcagataa cggggaaatg gagaccaact gccgcaaact ggtgtcggcg 6480
tgtctgcaat taggtaagaa ccccccttctc ctgcccgggt catcggacgg gaggccgcgc 6540
cacgtgaggg cggcaagagg gcactggccc tgcggcgagg ccccagcgag ggcgcttcc 6600
ccgaggggcc agcctgggca ggaaggaaat cagaaccaaa tcgccagtgg cctctccctg 6660
tggcggggcg gtggactagg aagcagcggc gcgctgtgta ccgaagcccc cagcctactc 6720
ctcccggctg gaaccgccgg caaccgggga ggcgcagaaa gagtacgcca tcctgccccg 6780
ggttgccaga gggtccgggg gacggggatg ccgtcagctc tttcctctaa ctgggtcttt 6840
gctctttgtc cctctttctc ctccggcctg cctcgcccct cccccctcctc ccgtccccgg 6900
ctcctttcgg ccgcggtccg ggacgcctct ctccgcacgt ggggcgggcg cgcgcgtggc 6960
ccaggcgtgc agccggcggc cgttgaatgt ctcttctcca aagactccga aatcaaaaag 7020
gtcgagttca cggactctcc tgagagccga aaagagcagc cagcagcaag ttcttcccgc 7080
ggcagcatcc tggcgccaat ggtaaggccg ggagggaagc gcaggccgcg cgctgggcac 7140
ccgcctccgg gactctgggc ctcgggcgaa agcgcaagaa ggcgaggccg ccagacctga 7200
cgcgcctgct gcttgaactt agacactccg cctctgggtg ggacgggaag cagccgtccc 7260
agggacgtta attcctttcc ccaaattata ctgcactcct gagacccaac acctctctct 7320
ccctctcgct cgctccctgt ggtctgatcc cctgcgcacg ctccagcaaa cctcggcgtc 7380
taggctggcg tggaaaagtg gtccaacagc gacctctgtc gctcgttata tcccgctgcg 7440
cgcagcacca ccagggccca ctcagtcact caggctctca gccacgcccc acccagactt 7500
gtgggtgcgc ggttcatcgc gggaggcaag caagggaaac ttgagttggc gaaggtttgc 7560
tttggctggt tgggggaggg gcgggggggcc gacaacatcc ctgaagagct ggagggcagc 7620
cactgtgctt agcagcccag ggtagaatgg aggtttgccc ctgtcgacgc gaacctgcct 7680
gaagtactgg ttgccaggcc ttgggttttg gcgatgtcct tgcttgattg gctggtttca 7740
ctctggagga atcgagggac attgccagag gaggtctaca ggcttatgta aaaagttaaa 7800
aagtctccaa cctacgccac aggcctttcc tgaattgaaa cttgttctat ggggcggagg 7860
gggggggtgta agggatgaga gagggaaaat gcctttcttt tcaaatacat ggaaaaaaac 7920
ccctcaaatt tactgttcct ctattttcct ggctccgtag taaataagtg cctagcctta 7980
ggaggctatt gacctttgat aatgtgagca gataaagccc cccccccccc cccacagccc 8040
tcggccccta accccctcttt tccggattaa agtgtaagaa tacaaatgta atatgggatg 8100
gagggggggcg atttgggacc ccggtcaaaa aaacaaaccg tattactaag aagaaaacaa 8160

aggtcttgca ttggagtttc cccgtgaatc tgagagaaaa tgatcatttg ttgaaatgaa 8220

gcgtctaaag cgatcctgtg cttcacgccc ggacactgca ctacaccgcc agtcgccctg 8280

ctgggccagt taaacgctca cttgtccggg attaacccca tggggttaaa tgggggcaat 8340

gcagagataa cgtcgcgtga tttctgtcat ttagattgtg ttaaattctt cttctgtttg 8400

ataatcggta gtaaaaataa attattagac cgtagtatgt ttgggatatg gttaaaaatc 8460

aagagcagcg atgacttctg gggagaatgc tttgcgcggg ctcagccctg gttccggcca 8520

gagtagagga gctccccaat ctcgcttcgc gcggggcggg ctcgcagtcg ccaagccgag 8580

gctgacattt cttattgtgc tgggagccag agagacgcaa aatgtctcct tcccccagtc 8640

cctaccccag gtttcctaga catggggaat gcattctgag gacaggtgga gaagcctacg 8700

gtaggatggg gtcctcgtag gtgagcagga aacggctaag agcagaggag ttctgcttgc 8760

gccagtcaca agccgcgcag gcgcccctgg tcgttcgctt ttgataacta gcacataaag 8820

aactagaaat aatgaatgat tgctttctta accattactt tcaggcccgc attgttttag 8880

tgcacgtgaa aggctcttcc cctacactca atatgtcttt ttctactttt tgaccgaaaa 8940

gaaaaattgc tgcctaaaca cgtttaatgc cattaattaa gaaaaggcat gtaatgggaa 9000

gaaatgctga aaatcttgat ttaattggct tttaaggaac tagtagacga caaaaaaaaa 9060

tcacacgagt gggcaaagct atagcactgc tgaaggatag agcacctatc cttccctgat 9120

tttaagttaa cttatggaat atccaaagtc ctggccacat cctgcttgta aaacaaaagg 9180

atttatttct tgtgtttttt taaagttttt ctttgctttt aaagagaaaa aaagttcaca 9240

atgacatatg acttcttcaa aaggccgtga tagtctatta cgctaccctc tccgcctctg 9300

cccccaacgc cgcccaaaaa taccatgtcc tcgttaaaga ctaaacgttc tgtataggca 9360

gagtccacct ctaagcagtc caggctctgc cttccttccc tagtgagtcc cattctcctt 9420

ggtatccatt gggcggatgc ccagcctgga tagaaccccg aaacgggggt agcacgagag 9480

cgactggaga cccctaaaag ccagaggttt gagagagggt ggacgcagct agcagaagat 9540

ggtgtagaag ccagctgaga acgacccccct agagcaaaga gacctttctt tggcttttct 9600

tgctttgggg gtcctgaaag gaactcataa aatggtcttc accctcagga ggaggacgga 9660

ctgaccctcc tctgtcactg gcttaaaaag tttcagggcg gcggctctgg gttccgtgct 9720

gaaatcggac tgcactgcag cccctttgga cctgacgcct ggctttgcgt cccgacaagg 9780

ggcgggtact tcccccggcc tcccccagga acgcattaat tgttaaatag ctttggccca 9840

gtggatgggc tgaaagtgtt cgacccaagt cgctggtgtg cacagacttc ctccctctgg 9900

gaggtgggct ccatggcccg ttgtggcacc cccagccgcg acacacacct tcacacgcgg 9960

cagcagctcg gtcccaacct tcttcgaagg acctgggtta accctggcgg ccttggcggc 10020

cgcagacccc tctccgccgc cccgccccccg cgcctctcat tcaatcagcg aatgtttgcg 10080
gagcatatgc tacgtggact cctaatgtac cccctgaaag caaataatac agttttcctc 10140
gccgtcatga agggatttta atcctaacat ggacaccagc gagatcagcc cagaccgtct 10200
ctagcaaaac gcaaaatggt ggtgcgtggg gtggtgacca aggtcctgag ccttgtcaga 10260
aagaagggga tgtgtagaga aaggtggaga actctagctg tggctagcgc ggaagggaca 10320
ggtgcttgcc gaagggggca tgaggcttga ggaaaaagca acgaaatagg ggtaaggaga 10380
gttttccatt ttctttcctc cgcccgacct ccgccatccc attctcccct cccctcaccc 10440
acccccccgcg ccaatcaaat ctgcagctca cttgaaaggt gccccgccgc gttgtggttt 10500
ttcacccccca ggggaaattg taccagttgt ccgaaagtag tcagtccctg cggatccctg 10560
cccgcaaaag tggtcttcac aggtcgcgtt cctcgctgct gactcggtac acaaagtttc 10620
ttaaggctgg ttcggctgtt attcctcacc gcccgctgct aatatatgca gcagttgtta 10680
gagcggctcg ggggaaaagg aaatgtataa cgaaagctta ttcgtgagca ggaatatact 10740
aatggaacaa tctgatgtct tcttaatcct atgtaaaaag ctttgtcgtc ttcctaatat 10800
tgactgaatg ggtaattaat ggctcttcat ctacgcgaat accctgtaat ccaagatagg 10860
caaaagacta caagcccaag gtaagaagac aaaaggccca actgcagcgg cgtttgcccg 10920
cctcctaacc cccagggtct ctgactatga gagtcttctc tcatacgaga aaaagcagga 10980
gtgggagaat atatacctat catttcgggg ctagacttca ccgcagcacc tgaccaccca 11040
gctcagtctt ctgttactct gtctttccac ctccagtccg tctccctgca tttttttct 11100
tcttaactcc tctaggatga cctcctacca ccaccgccat tatggtccta ataacctttc 11160
cccctaaacc ccacatcttt cacttcagca accaatgagg ctgttttctg atccaggagg 11220
agattctttt cttttagaac cccatgcgta cagtctttga gaactaaagt agttggtagg 11280
ggaggaagaa attaatagaa agggagagag catacagaat tgtgtgtgta tgttaaagag 11340
cgaccaggaa tgacagagtc cacttctttg tgaggatctg acgggaagag tgtccaacac 11400
tctacagcat gtttttaaca ggctgacatt ttaatttaaa cctttagaag taatatatta 11460
cttgggttac tacaatgagg tgggttcctt tttttttgtc agctgacagc ttttaaaata 11520
ttatttcgct agggaaataa aagcttcatc tcagaattat aggtgggtat ttttggattt 11580
aggtgattta tggtcaccat gacaactaat gctgaatgtt agctaccaac atgtctggga 11640
gagagaaaac agaaagaagg gagagcaaaa gaaatagaaa agggagatgg acataacctg 11700
gagagggaag aaaagagaaa aagaggagga cagatgagtg tttaatccaa ctgtggttta 11760
aaaaagtggc gggggggtgg gatttcattt accccctagc aatttctttc tttcaggacg 11820
tggatatata tgttaaattt aatattcaac ctttccttcc ctttctccca aacacatgtg 11880
ctataccatt cttccttatt taacttagtt cggcaagtag ttgctttctg gagactcagt 11940

gacacctagg aaaactgtgg cagtaacatg caaatgtgag gaagcattaa cagcatgttc 12000

gttgagtgat tttagcaaat gccccctcct ctaatctctt tctcttctct tccccaggcc 12060

actgtgaggt ggtaactccc actgccatct gaacactgtt cccccaggta gttaccccaa 12120

atctcaaatg gttgagcagc tagagctgtg ggttggaaaa atgggtacca tttgcaggga 12180

cccagagagg gtggctgttg ctcaatatat ctacagattc caattcagaa aataatcttc 12240

cccttttgac aagccagagc tttttaaatt ccatttagga aatggggaaa aggacagcta 12300

cagtgaagct tttaatttct gggttatttg gttccatagc tatgaggggt ggtgggtagt 12360

ggactgtttt cagcttggtt tgtatgcaga gaaaagccag atattggagg gggtggggca 12420

cttctcgggc aggatgcaag gtctccatct gacctctgcg ccttaccagg agctcacaca 12480

ctcacgccca tcacgtggct ccaagctgag tccaggcggg cctcgtcctt gagctagctt 12540

gggcagggca ggactcccac ccgtctaagg cctaacagct cagggagatg tctaattaag 12600

ccatcttctg ggtgaactct gaagacagac tctttccaaa agtcagagat cacttggttg 12660

agtcccaggc cagattgata cggagagttt ggcggcacag cccaaccgct cactgccatg 12720

gccagaggga ctttgcacaa ctaacactga agagtgtgaa attaaataag actttaagac 12780

tggtaatcgg tggcaaatac cagcacaaaa cacggctgac cccatgtcac acatttccct 12840

cctctagggt ctttctttga aagaataagc aagaaacccc aatcgagaca acccctgatg 12900

tctcccagac tcaaaacctc acgccggcac tgggctttcc ttctttgccc cacgtgagcc 12960

atgcagcacc tctagtttcc ctaccaggat cccaccaatg ttctccgtat tggaaatttc 13020

tgtgttagag gctgaactca tagtaatttc taaaaccaat taagaagaac ttagccagag 13080

gtcacagtaa tgctggaatc acaaaatgca taagatttat tttcttctgg cccctttctc 13140

atccatgctg cctatgcctg tgcacccaca agtcttatgt acattaaatc tccaaaatca 13200

accaccacca tgccacagag ttttcactgg acagtgtttc ttagttccca ccacatacct 13260

ccttttcccc atgcagactt atcatgttgg tgtcctgcca cacagggggc ctgagaagaa 13320

tgtcacccaa tcgccgctgc tgtgagtgtg taaagtgatt aggagattag gagaatgttg 13380

aaactcctgc tggaaaaatg caaagaaaat cctcactttg agtcagttgt ttacagagcc 13440

agtgtgtgtg cgtgtgtgtg tctgtaatat aaaatggatg tgaatatata tatacaaata 13500

gatatggttt tgcttttact ttaatctgaa ttatctagat aattgtcttt attcaccact 13560

tgattcaatg gtccacacaa attaggacac cttatctttt taggcatcta ggctgctgct 13620

gatccccagt gcttccaaca tctcgcacac gttggcacta tgaggagcag tcacgtgccc 13680

ttgggttttt caaccacttt ggaggctgat tgaggtcctt catacatgta tatttgtcgt 13740

gatgaaagtt ccatcggtag agtggagcca ccagagcttt tatcaaaatt ctgtgggttt 13800

atgagagatg ggtttagaaa tctatatggc tctgtggggc ttctcggctt tctaaaataa 13860

ggcattaaca cccaagcttc caaaaatatt tgcagctctg gggtttgaat cttgaaaaac 13920

aaggagtgag gggctgtgta tactaactac agtggagatt tttttcattc tttaatgtga 13980

tggagtccct tcatgaaatg aagctttaag gggcatggta ttgtggggac cacagctatt 14040

ctgaggttta aaagaagaaa ctggaatatg attagtaaac acattcagca gaaaagagct 14100

ggattcttat tgacttagtt ataggtcatc ggctggcagt gcaatgggag gaaatattta 14160

ctttacacat atactttatg atcttggggg aattagagga aattcaataa gaaaacggct 14220

agaaacattt aaaaccctta tttaaaagac ttaagcaaat tagagtctta tcagattaaa 14280

aaccactaca aatgtaagag cattgtcttc agtgaaacgc tgtggggtct gagaaggaga 14340

ttctccgcca aatctccggg ataaaatgcg tcatttaagc accagataat gagcagaatg 14400

taaattaatt taaccttctt taccaacagg ctgctagtgt aatgtgtata atttagtgat 14460

aagattgcag gacctaatat agctggatgt atgagcctca gctaatgcag acctgtcaca 14520

tgaggatgtg ttttactctg agcaggtgtc tgtatgtgtg gaatggggta aagtggaata 14580

aaaggttaaa agcagaaatg ctgatttaaa gcttactatg aagaaattcc tcccttgcag 14640

ctaaattatt cttaaagtgg gatgatactg gtgaagaaag actgaaaaac aattctcatg 14700

tgcgtctttg gactgcaagt ttaaaatggg gaggagttgc agatagggtt tgggggtggt 14760

cagggcaaag gagagacaca taagttgcaa atatatttgt agtctgcttc atccactttg 14820

ttccacatcg aataagtttc ccaatcttgt gaacaaggac aaggagggag tgttttaaag 14880

atacttcatg ctggcattgc aaatcattga ctgtaatgtc aaacaaatac acattcagag 14940

atgataacac taactccata gtaaaacaat cgcccatgca gaaacccaga ggagattagt 15000

ttgtcctctc cagctgacct atgctggggg acaaaaggac tttcaaaaat tattttgaat 15060

atgtttggat ttctttcttt aatttctttg gaaattaaat ttgcttggaa acagtgctat 15120

aaagagttga tgtctccaaa ggtgattttt tttgttttat ataaataagg ttttgctttt 15180

gctagttgag cgcagttcta ggcttttcgc ccttagctca cacacacccc ttctgcctgc 15240

ttggacttta atggctcaag acagccttga gctcactggg aaaagaaaat gactgttaaa 15300

aattatcctt gaaattggtt atttggcaac attcttaatt gtatggaaat tcattaaggc 15360

atatttcata tataattagc tcaaggttgt tgattctaca ggctttatgg atttaaatct 15420

gattgataat aaagtaaaca agagagtcga atttaaagcg tggctctctc gggttaggac 15480

gagcttaata cagtgtacaa ggaatttgaa agatctagga tatgtgtctt aatcaacgtt 15540

aagtagaatg gataagcttt cagcattttg aaaacgctgg gttagggttt ctcttctatt 15600

gtgtgttttc tgtctgggga ctaataagca tcacagagaa cgtgatctga ggcgactttt 15660

tattcttgta taaatccaga gtgaaccacc aaacagttgt tcgtttaaag tcaaggtaat 15720

tttcttttga cgggtccatt tgcttctcga tttctaattt attagcctgc cttttcaggg 15780
ctctgtcttc tttgcaatta aagcttcttc agattagcgc agcattcact tgacaggctg 15840
tttggaaaat ttaagatcgg agaggtgatt tgttgctgtt tttcaaattt tctagtttta 15900
agtaacgtgt ctccttttta tatggggtgg gggattggaa atggatgtag tgagacacaa 15960
agagtgggtg tcttgttgat ccttgtacct ttctcttctt gaccattcca ctctcttctc 16020
ccaagccttc gactcctagc ctcatctctt cacctttggg ttcgtactaa aagccggatc 16080
gccttgggct gggcaggagc tgaattcccg ggagcttgcc tgtgtagacc cagtgcgcac 16140
ggcgaggcag tagcccggcc ccgcactgct gataggtgca ggcaggacag tccctccacc 16200
gcggctcggg gcgtcctgat tggtgcggag ccacgtcagt cgcacccgga gaagggtctg 16260
ggaggaggcg gaggcggaga gggctgggga gggccgcggc ggagtgacgt ctcggcacca 16320
ggaagcccgc ctctggtttt aagatgttag gccaacaggg aagcgcggag ccgcagatct 16380
ggtccgtcgc tcgcctgggt gcctggagct gagctgcggc aaggcccggc tcctgttcga 16440
ccgcccgagg ggtgtgcgtg tgcgcgttgc ggagggtgcg ctcagagggc cgcgtcgtgg 16500
ctgcagcggc tgctgccgcc gcaggggatc taatatcacc tacctgtccc tgtcactctt 16560
gacacttctc tgtcagggct gccgcgtggg ggggggcggg cagagcgcgg tcggcgttag 16620
ctttccttat tggaggggtt cttgggggag ggagggagag aagaaggggg tctttgccca 16680
ctcttgtttc gctttggagc ttggaagcct gctccctaaa gacgctctga gtggtgccct 16740
tctgcccaca tcccatgtct tcgtttgccc gctgactttc cgtctccgga ctttttcgct 16800
tgagccttcc ggaggagacg gggcagcttt ggcttgagaa ctcggcgggg gttgcgtccc 16860
ctggctctcc ccgcagcggg gaaactccgc gcctagagcg cgacccggag cgggcagcgg 16920
cggctacggg ggctcggcgg ggcagtagcc aaggactagt agagcgtcgc gctccctcgt 16980
ccatgaactg catgaaaggc ccgcttcact tggagcaccg agcagcgggg accaagctgt 17040
cggccgtctc ctcatcttcc tgtcaccatc cccagccgtt agccatggct tcggttctgg 17100
ctccggtcag ccccggtcgc tggactcctc caagcacagg ctggaggtgc acaccatctc 17160
cgacacctcc agcccggagg ccgcaggtaa ggcgccgcgc cgccctgcag acattcccgc 17220
ttagctgctc tgcgccaccc gctccctctc gccccaagga agtcagcccc tccgcgggga 17280
ggcgtggtgg gagtggtcgt tcgcctggct ccccgcagaa cttccgggag ccggaatttt 17340
gactaccccg catcccttta gttctccctc gaccggcccg gctcctgggg cgctaagggc 17400
gcgagcaatt ctgccgccct ctctattcgt accctggcct cccttctgtt tcctgggtca 17460
caaaaatccc agcatcttga ttcgaggacc ttcagaggcc gccgacctct gtccctgttt 17520
tcctctcggc tttcagctcc cgaggagctc cactcgttag gaaattgcct gaaaccactc 17580

agaaatgccc ttcgcgaaga ggcatttttt ttttttttg ggaaagggcc ggcgaacttc 17640

ggtgcccaac cgaatcccca catcttttcc tagccttccc aaaccgcatg gaaatctgag 17700

ctttctgcga gggggagggg ggtctgtaaa ccacgcgcgt gtgcgcgtcc caggagattt 17760

ggtgtgtctg cgcagaggtg tataaatata cttgaaagca caggctataa aagtgaatgt 17820

gccgctgcag tgagataaac atgtaaataa aacgtgcggc gctgggggag gggaggaaat 17880

ggggcgcgga cacccacact tgcgacctgc acaccccaca ggcgcagcgc tcctcgcggc 17940

ccggagccgc cgcgcgcacc ctcctccggc gccaggcagc ccagctcttc cacggcttct 18000

gccgccggtc cagttggcgt ccgcgttgca ggtgggcatg ctgacgggaa agtgtgtgtg 18060

tttcgttttc agagaaagat aaaagccagc aggggaagaa tgaggacgtg ggcgccgagg 18120

acccgtctaa gaagaagcgg caaaggcggc agcggactca ctttaccagc cagcagctcc 18180

aggagctgga ggccactttc cagaggaacc gctacccgga catgtccaca cgcgaagaaa 18240

tcgctgtgtg gaccaacctt acggaagccc gagtccgggt aggagccagc acggagtctg 18300

ggagggatgg ggggaggatg ttgtggaggt acaggccaag tagaccagga gagaatgtgg 18360

aaggcagcgc cgcctgggag ggcgccggtg gggcgcagct ttgcaaaggc agaaggcctc 18420

gcggcggcct ggttgcgaga ttacagttcc ctctccgagg ccgacaggac tgccgccctg 18480

gctcaggctc ccagagcggc accggctcac tgccccgcca tcccgcgatc tcacgagctg 18540

ggctgcatgg gcaatcccct gcacaggaca ttgtgttcct ggcttgcagt tgccagagca 18600

gagctaataa aatccctacc aggccaagag ccgcgaacag gctccaacct gtgagccttt 18660

aacaaggaaa acccgccaga gacacggaag agttggccct ccctgggaaa cctttgtccc 18720

ggccctggcc cagctttttc cctcctgggc tcgcgcttct tacaccttct ttacggttgt 18780

ttcggccatt caggtctctc ccacacaccc tatttcctag ttttgtgatc tccgggagca 18840

aagttttaat acacaactac tagtcctctt agaaggagaa agaaaaaaag aagaaagact 18900

tttctgcttg gtttatttat cttctctcag gagttgaact ctggaaattg aaactcacac 18960

cccctcttct aaattataat catagttttg taaaaagggc ttaccttaac tttgtagcaa 19020

atctgtactt tatggattgg caaaaatgag ctcaaataaa taacccaata gcaacgtcct 19080

ggtttatgct ggtcggtgga agattccaaa tttgttagga ttctggaagc agaaaacaga 19140

atcaagcaaa tcaagcggca tccagaggct ttgctgttaa aaaaaaaaaa ttaagtgctc 19200

tgggtagaaa aaataaagcc cccggttaga gcagagcaca ctaaaagaag aaaaccacga 19260

ttcttcgaat aatgaccaaa atgctctccc ctatcagagg gaatgaagac actctctggg 19320

tggcatttgt gcaacgcatg acgctatgct ggtggataaa aggccgggaa gaagttgaaa 19380

atggttttag tttaactgtc cacagccaga cctgggtcct gggcggcgtg gttttgatca 19440

aggtcagtct ttcattacct ctcttgcaca tcaagggaac gggcctctca cgtactcttc 19500

tcgcctgagc aaagtttaga tggcctaggg tagaaatggc aagtaattaa agacagagtc 19560
tatgggtttt ctgggatcct tcgaacaacg ccctcccacc ccgcccgcta ttccgcagct 19620
ccaccatagt gatttgtagc cgcggcgctg ggctctcctt gcagctgcct ctccttccag 19680
ggcggctgct tgtcgagcca agtgggagtg aggcgtgctt tttatagcag tcgggtgcaa 19740
agaggaaggg ggataaaaag gaaatcaaga atgaaaggaa aaagagaaaa agcggattac 19800
acggtctggg cccggcggag atgtgtaatg tgaaacatca ctggtgtcag ctcggatatc 19860
tcaggccagg cctctctcca atacacaaaa gccgccgtct ggggcgacag ggaggcccga 19920
tgtggattgg gatcggggtt gcggctgggc caccggacac gggtggaagc cggccggcgt 19980
gggtggccgc ctgcaaagcc aaacgacccg gctgggcctg gcgcgcggac aggcctgtgg 20040
tgggcttagg gtaaagaaga ggcagagcga aagaaggggg aatctccaaa actacccttt 20100
ccgggttccc ggagtttaat atgccaagct cctggagtta acgagctgac gaagaggtgg 20160
tcttttgctc tttatttggt tgttttgcta ggcgagaaag agtgttggcg gcctagtccc 20220
tgttaaggga gcacgtacca gggggtgggg gacgacagtg gaggtcaggg aaggaaggga 20280
ggaattgcgt gggagaaaga gcgatcctct agtgcccttc cagccccttc tcctcatccg 20340
tgggtctgtg gctttggaat ggaagcaagt ttgcaaggtg ccccgggaag ggttggaaaa 20400
gcctgctgcc ccgcgtttgt tttacattaa gtgtttttgg acctggagaa acgcctggct 20460
gagtgatcaa accgtccgca ggtctccatg cgttcggctg aggtttgtgg cgtagctccg 20520
agtcccagct cgcaggccag agcagaccag gtctcctgcg cttggtggag acccgggcca 20580
gtaactgaaa gctggccctg gtatcttggt gtgcagggcg gtgcagtgaa gcgaggccag 20640
ggtgtgtgag tgcgctagcg tgtgtgtcgg gggaaggcgg gggttggcct ccgatggaag 20700
ttttagtaat ctgcactgtg gcatctgttt gctcccttgc cccaaccgcc cccaggtttg 20760
gttcaagaat cgtcgggcca aatggagaaa gagggagcgc aaccagcagg ccgagctatg 20820
caagaatggc ttcgggccgc agttcaatgg gctcatgcag ccctacgacg acatgtaccc 20880
aggctattcc tacaacaact gggccgccaa gggccttaca tccgcctccc tatccaccaa 20940
gagcttcccc ttcttcaact ctatgaacgt caacccccctg tcatcacaga gcatgttttc 21000
cccacccaac tctatctcgt ccatgagcat gtcgtccagc atggtgccct cagcagtgac 21060
aggcgtcccg ggctccagtc tcaacagcct gaataacttg aacaacctga gtagcccgtc 21120
gctgaattcc gcggtgccga cgcctgcctg tccttacgcg ccgccgactc ctccgtatgt 21180
ttatagggac acgtgtaact cgagcctggc cagcctgaga ctgaaagcaa agcagcactc 21240
cagcttcggc tacgccagcg tgcagaaacc ggcctccaac ctgagtgctt gccagtatgc 21300
agtggaccgg cccgtgtgag ccgcacccac agcgccggga tcctaggacc ttgccggatg 21360

gggcaactcc gcccttgaaa gactgggaat tatgctagaa ggtcgtgggc actaaagaaa 21420

gggagagaaa gagaagctat atagagaaaa ggaaaccact gaatcaaaga gagagctcct 21480

ttgatttcaa agggatgtcc tcagtgtctg acatctttca ctacaagtat ttctaacagt 21540

tgcaaggaca catacacaaa caaatgtttt gactggatat gacattttaa cattactata 21600

agcttgttat tttttaagtt tagcattgtt aacatttaaa tgactgaaag gatgtatata 21660

tatcgaaatg tcaaattaat tttataaaag cagttgttag taatatcaca acagtgtttt 21720

taaaggttag gctttaaaat aaagcatgtt atacagaagc gattaggatt tttcgcttgc 21780

gagcaaggga gtgtatatac taaatgccac actgtatgtt tctaacatat tattattatt 21840

ataaaaaatg tgtgaatatc agttttagaa tagtttgtgt ggtggatgca atgatgtttc 21900

tgaaactgct atgtacaacc taccctgtgt ataacatttc gtacaatatt attgttttac 21960

ttttcagcaa atatgaaaca aatgtgtttt attttcatgg gagtaaaata tactgcatac 22020

<210> 3
<211> 144
<212> DNA
<213> Artificial Sequence

<220>
<223> Bisulfite-converted PITX 2 sequence with a fully methylated status to which sequence a Taqman probe for methylated sequences will bind

<400> 3
gtaggggagg gaagtagatg ttagcgggtc gaagagtcgg gagtcggagt cgggagagcg 60

aaaggagagg ggatttggcg gggtatttag gagttaatcg aggagtagga gtacggattt 120

ttattgtgga aaggaggatt agaa 144

<210> 4
<211> 144
<212> DNA
<213> Artificial Sequence

<220>
<223> Bisulfite-converted PITX 2 sequence with an unmethylated status to which sequence a Taqman probe for unmethylated sequences will bind

<400> 4
gtaggggagg gaagtagatg ttagtgggtt gaagagttgg gagttggagt tgggagagtg 60

aaaggagagg ggatttggtg gggtatttag gagttaattg aggagtagga gtatggattt 120

ttattgtgga aaggaggatt agaa 144

<210> 5
<211> 144
<212> DNA
<213> Homo sapiens

```
<400>  5
gcaggggagg gaagcagatg ccagcgggcc gaagagtcgg gagccggagc cgggagagcg       60

aaaggagagg ggacctggcg gggcacttag gagccaaccg aggagcagga gcacggactc      120

ccactgtgga aaggaggacc agaa                                              144


<210>  6
<211>  24
<212>  DNA
<213>  Artificial Sequence

<220>
<223>  PITX2 reverse primer (PITX2-R)

<400>  6
ttctaatcct cctttccaca ataa                                              24


<210>  7
<211>  22
<212>  DNA
<213>  Artificial Sequence

<220>
<223>  PITX2 forward primer (PITX2-F)

<400>  7
gtaggggagg gaagtagatg tt                                                22


<210>  8
<211>  20
<212>  DNA
<213>  Artificial Sequence

<220>
<223>  PITX2 probe for methylated sequences (PITX2-Pm)

<400>  8
agtcggagtc gggagagcga                                                   20


<210>  9
<211>  27
<212>  DNA
<213>  Artificial Sequence

<220>
<223>  PITX2 probe for unmethylated sequences (PITX2-Pu)

<400>  9
agttggagtt gggagagtga aaggaga                                           27
```